# EUROPEAN PATENT SPECIFICATION

(11) **EP 0 487 541 B1**
(45) Date of publication and mention of the grant of the patent: **31.08.1994**
(21) Application number: 90910597.5
(22) Date of filing: 05.07.1990
(51) Int. Cl.: C12N 15/31, C12N 15/55, C12N 9/20

(54) **DNA ENCODING A LIPASE AND A LIPASE MODULATOR**
FUER LIPASE KODIERENDE DNS UND LIPASEMODULATOR
ADN ENCODANT UNE LIPASE ET UN MODULATEUR DE LIPASE

(30) Priority: 07.07.1989 DK 3368/89
(43) Date of publication of application: 03.06.1992
(73) Proprietor: NOVO NORDISK A/S, 2880 Bagsvaerd (DK)
(72) Inventor: JORGENSEN, Steen, Troels, DK-3450 Allerod (DK)
(74) Representative: Thalsoe-Madsen, Kine Birgit
(86) International application number: DK9000170
(87) International publication number: WO9100908

(56) References cited:
- EP-A- 0 214 761
- EP-A- 0 318 775
- EP-A- 0 331 376
- WO-A-89/01032

## Description

### FIELD OF INVENTION

The present invention relates to a DNA sequence encoding a lipase, a DNA sequence the product of which affects the production of the lipase, expression vectors and host cells comprising these sequences, and a method of producing lipase by cultivating the host cells.

### BACKGROUND OF THE INVENTION

Lipases are enzymes which catalyze the hydrolysis of ester bonds in triglycerides resulting in the formation of diglycerides, monoglycerides, glycerine and free fatty acids. Some lipases also catalyze other reactions involving ester bonds such as synthesis of ester bonds or transesterification reactions. Lipases are produced by a wide variety of different organisms. Microbial lipases in particular are of considerable practical utility for a variety of purposes where lipolysis of fats is desired, e.g. in the food industry and in detergents.

One particular lipase which has been found to be particularly advantageous for inclusion in a detergent for the removal of fatty stains or soiling from fabrics is a lipase produced by strains of Pseudomonas cepacia. In EP 214 761 (to Novo Industri A/S), this lipase is disclosed as a lipase which is active at a temperature below 60°C, which is important as most present-day fabrics are washed at temperatures below 60°C.

Another important Pseudomonas cepacia lipase for use as a detergent additive is the one disclosed in WO 89/01032 (to Novo Industri A/S) as a positionally non-specific lipase, i.e. one which is able to react with all three fatty acyl groups of a triglyceride.

In order to facilitate Pseudomonas cepacia lipase production, it may be advantageous to employ recombinant DNA techniques, for instance in order to optimize lipase expression by introducing a stronger promoter from which the DNA sequence encoding the enzyme is expressed or by introducing more efficient ribosome binding sites or signal peptide coding sequences, or in order to select a host organism for the production of the enzyme which is easier to cultivate (e.g. in terms of its being a standard production organism such as E.coli, B. subtilis or the like) or which results in higher lipase yields. As described below, such approaches will sometimes fail to yield the expected results, e.g. in cases where one or more genes in addition to the structural gene coding for the protein in question, play some part in the production of the gene product (examples of such genes are the Bacillus sac (Honjo, M., et al. (1987), Journal of Biotechnology, 6:191-204) and iep (Tanaka, T., Kawata, M. (1988), Journal of Bacteriology, 170:3593-3600) genes, and genes required for the production of Klebsiella pullulanase and E.coli hemolysin).

The cloning of a lipase gene from another Pseudomonas species, Pseudomonas fragi, is known from, e.g., S. Aoyama et al., FEBS Letters 242(1), December 1988, pp. 36-40, and W. Kugimiya et al., Biochem. Biophys. Res. Comm. 141(1), November 26, 1986, pp. 185-190. However, the lipase produced by P.fragi differs from that of P.cepacia in its amino acid sequence, and in these publications, there is no indication that one or more additional genes may be required in order to achieve a significant lipase production in a host organism.

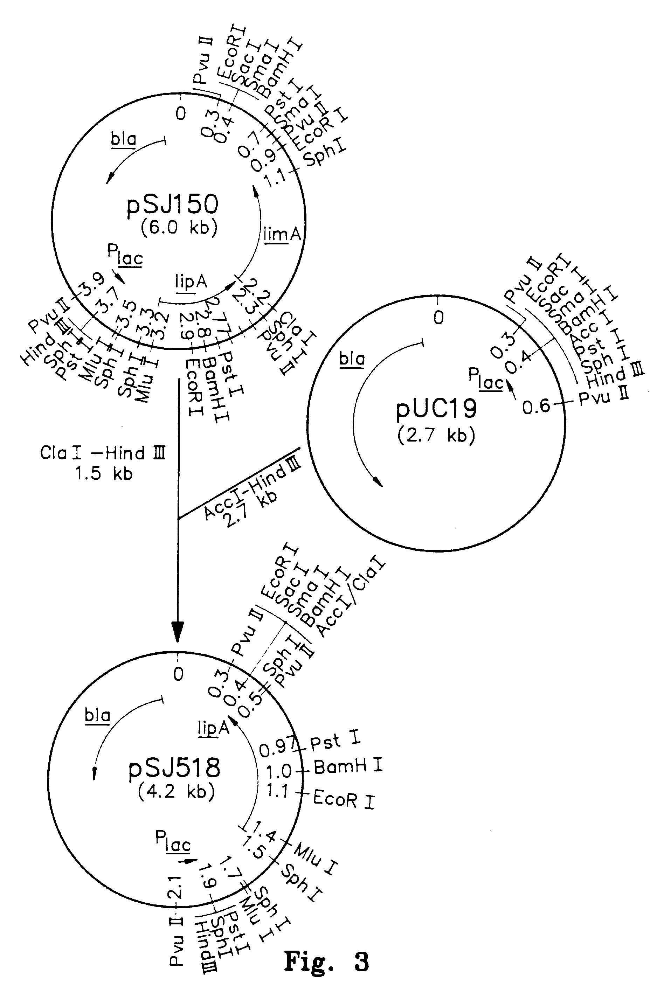

EP 331 376 discloses a recombinant DNA encoding a Pseudomonas cepacia lipase as well as a protein participating in the production of the lipase. The amino acid sequence of said protein is shown in Fig. 3B. There is, however, no indication that the gene encoding this protein may also be functional in a heterologous host organism.

### SUMMARY OF THE INVENTION

The present inventor has isolated and cloned a gene encoding a Pseudomonas cepacia lipase and identified a gene the expression of which is important to achieve production of the lipase in significant yields.

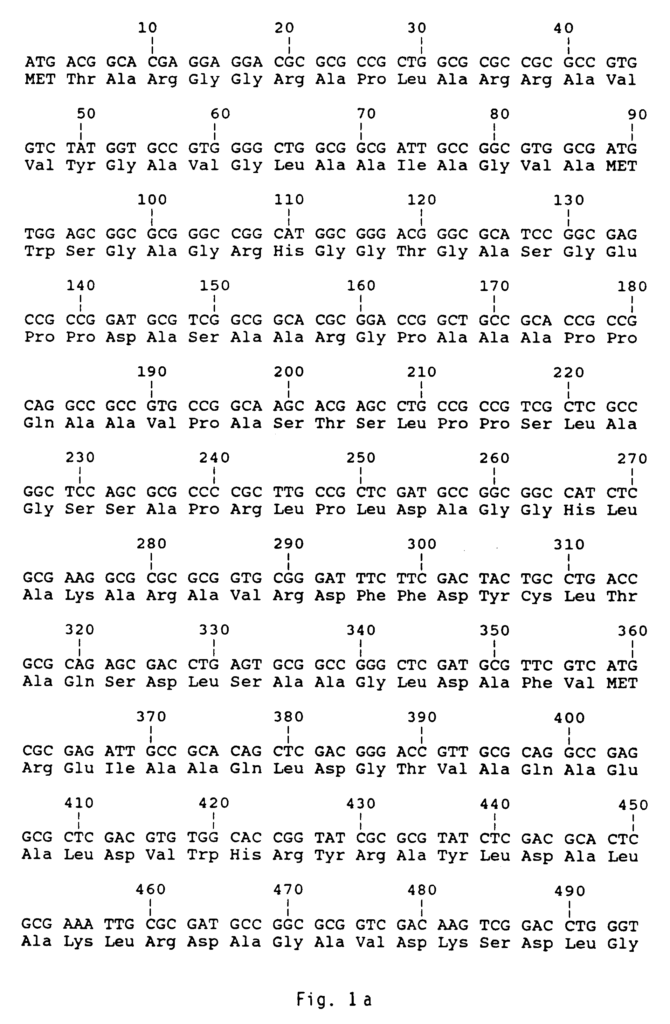

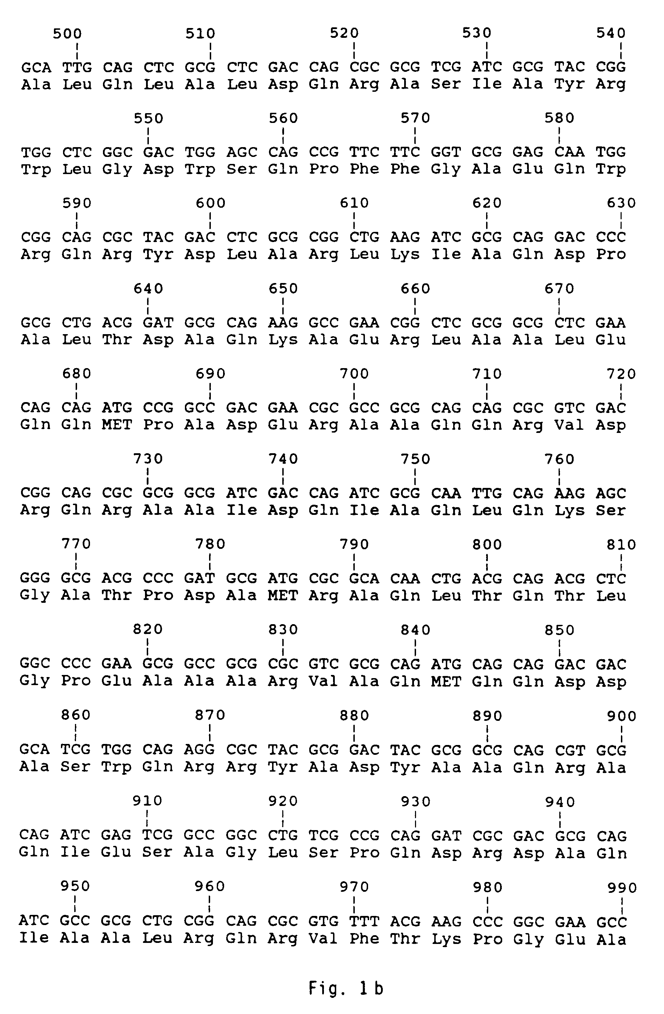

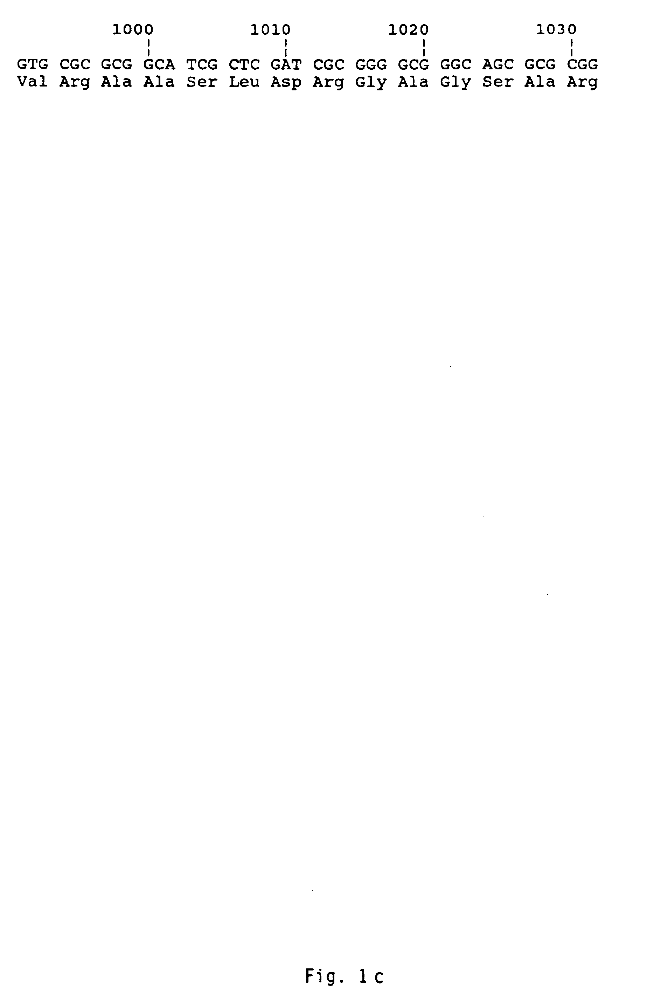

Accordingly, in a first aspect, the present invention relates to a DNA construct which comprises a DNA sequence as shown in the appended Fig. 1A-C, encoding a factor which acts in trans as a modulator of the production of a Pseudomonas cepacia lipase, or a modification of said DNA sequence encoding a functional analogue of the P. cepacia lipase modulating factor, the P. cepacia lipase modulating factor or functional analogue thereof being capable of modulating lipase production when the P. cepacia lipase is expressed using expression signals which are heterologous to Pseudomonas and/or when the P. cepacia lipase is expressed in a heterologous host cell.

In the course of the research leading to the present invention, it was surprisingly found that a region downstream of the DNA sequence coding for the lipase (as present on the chromosome of P. cepacia) has a pronounced beneficial effect on the production of the lipase. It was experimentally established (among other things, by inserting this region on a another plasmid than that carrying the DNA sequence encoding the lipase) that the region does not merely provide a site on the DNA sequence (such as a promoter, terminator, enhancer, or the like) which is required for lipase expression, but that the region includes a gene encoding a factor which is able to affect the production of the lipase even when the DNA sequences coding for the two products are not located on the same vector. The lipase modulating factor may be an RNA molecule or a polypeptide, but is most likely a polypeptide (cf. Example 3 below).

In the following, the DNA sequence encoding the lipase modulating factor will usually be referred to as the "lim" gene, while the modulating factor will usually be referred to as the "Lim" factor.

It has surprisingly been found that expression of the lim gene exerts a beneficial effect on lipase production even when the lipase is expressed using expression signals which are heterologous to those of Pseudomonas (e.g. a promoter, ribosome binding site and signal peptide-coding region from a Bacillus α-amylase gene) or when the host organism is not Pseudomonas. Therefore, the Lim factor does not act on the initiation of either transcription or translation. However, the precise function of the Lim factor has yet to be elucidated.

The term "functional analogue" is understood to indicate a factor which has a similar lipase-production modulating function to that of the P.cepacia Lim factor and which is derived from another organism than P.cepacia, or which is a derivative of the P.cepacia Lim factor produced by modifying the DNA sequence in a manner resulting in addition, substitution, insertion or deletion of one or more amino acids in the native sequence. There is reason to believe, however, that such modifications of the native sequence should not be too extensive as the modulating function of the Lim factor may otherwise be lost. Apart from this, a functional analogue may be a homologous polypeptide (i.e. one encoded by DNA which hybridizes to the same probe as the DNA coding for the Lim factor under certain specified conditions [e.g. presoaking in 5xSSC and prehybridizing for 1h at ∼40°C in a solution of 20% formamide, 5xDenhardt's solution, 50mM sodium phosphate, pH 6.8, and 50µg of denatured sonicated calf thymus DNA, followed by hybridization in the same solution supplemented with 100µM ATP for 18h at ∼40°C]), or which is reactive with antibodies raised or directed against the Lim factor. When the term "Lim factor" is used in the following description, this is intended implicitly to include such functional analogues as well as the native P.cepacia lipase modulating factor.

### DETAILED DISCLOSURE OF THE INVENTION

The DNA construct of the invention comprising the DNA sequence encoding the Lim factor may be of genomic or cDNA origin, for instance obtained by preparing a genomic or cDNA library of P.cepacia and screening for DNA sequences coding for all or part of the Lim factor by hybridization using synthetic oligonucleotide probes in accordance with standard techniques (cf. Sambrook et al., Molecular Cloning: A Laboratory Manual, Cold Spring Harbor, 1982).

The DNA sequence encoding the Lim factor may be any one obtained from a lipase-producing P.cepacia strain. Examples of such strains are the ones deposited in the Deutsche Sammlung von Mikroorganismen in connection with the invention disclosed in EP 214 761, with the deposit numbers DSM 3333-3337 and DSM 3401, as well as the strain deposited in the Deutsche Sammlung von Mikroorganismen in connection with the invention disclosed in WO 89/01032, with the deposit number DSM 3959. It is, however, envisaged that a DNA sequence equivalent to the lim gene may also be derived from another lipase-producing organism. Such a gene may be screened for by hybridization using probes that hybridize to DNA encoding the Lim factor as described above or selected by the reactivity of its gene product with the same antibodies as the Lim factor or identified by screening for the ability to confer lipase production to strains containing the lipase gene but not the lim gene.

The DNA construct of the invention encoding the Lim factor may also be prepared synthetically by established standard methods, e.g. the phosphoamidite method described by S.L. Beaucage and M.H. Caruthers, Tetrahedron Letters 22, 1981, pp. 1859-1869, or the method described by Matthes et al., EMBO Journal 3, 1984, pp. 801-805. According to the phosphoamidite method, oligonucleotides are synthesized, e.g. in an automatic DNA synthesizer, purified, ligated, and cloned in an appropriate vector.

Finally, the DNA construct may be of mixed synthetic and genomic, mixed synthetic and cDNA or mixed genomic and cDNA origin prepared by ligating fragments of synthetic, genomic or cDNA origin (as appropriate), the fragments corresponding to various parts of the entire DNA construct, in accordance with standard techniques.

Preferred DNA constructs encoding the Lim factor are those comprising a DNA sequence derived from the chromosome of P.cepacia, in particular those wherein the DNA sequence is located on the P. cepacia chromosome downstream of the sequence encoding a ∼33 kD lipase.

Examples of suitable modifications of the DNA sequence are nucleotide substitutions which do not give rise to another sequence of the Lim factor, but which may correspond to the codon usage of the host organism into which the DNA construct is introduced or nucleotide substitutions which do give rise to a different sequence and therefore, possibly, a different structure without, however, impairing the properties of either the lipase or the Lim factor. Other examples of possible modifications are insertion of one or more nucleotides into the sequence, addition of one or more nucleotides at either end of the sequence and deletion of one or more nucleotides at either end of or within the sequence.

For some purposes, it may be convenient to provide a DNA sequence encoding a Pseudomonas cepacia lipase on the same DNA construct as the DNA sequence encoding the Lim factor. Thus, the present invention further relates to a DNA construct which comprises a first DNA sequence encoding a P. cepacia lipase or a derivative thereof and a second DNA sequence encoding the P. cepacia lipase modulating factor.

In the present context, the term "derivative" is intended to indicate a protein with lipolytic activity which is derived from the native lipase by suitably modifying the DNA sequence coding for the native lipase, resulting in the addition of one or more amino acids to either or both the C- and N-terminal end of the the native protein, substitution of one or more amino acids at one or a number of different sites in the native amino acid sequence, deletion of one or more amino acids at either or both ends of the native protein or at one or more sites in the amino acid sequence, or insertion of one or more amino acids at one or more sites in the native amino acid sequence. Such modifications of DNA coding for native proteins are well known and widely practised in the art.

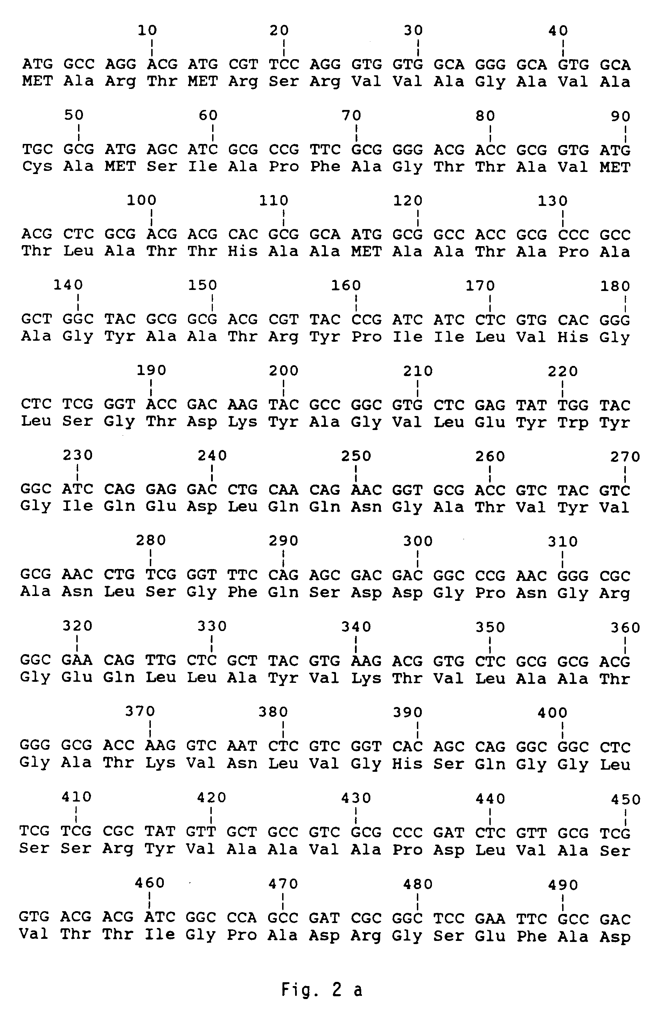

Preferred DNA constructs encoding the P. cepacia lipase are those comprising a DNA sequence derived from P. cepacia, DSM 3959, encoding a lipase of ∼33 kD which in nature is present on the chromosome of P. cepacia. A particularly preferred DNA construct encoding the lipase is one wherein the DNA sequence encoding the lipase is the one shown in Fig. 2 A-C appended hereto or a modification thereof encoding a derivative of the P. cepacia lipase as defined above.

In one embodiment of the DNA construct of the invention, the lipase DNA sequence may be located upstream of the sequence encoding the Lim factor, and in another embodiment, the DNA sequence encoding the lipase may be located downstream of the sequence encoding the Lim factor. In either case, the DNA sequences may be located relative to each other in such a way that they are transcribed from the same promoter under conditions permitting expression of the lipase and Lim factor when the DNA construct is present in a host cell. In a particular embodiment, the first DNA sequence encoding the lipase and/or the second DNA sequence encoding the Lim factor may further comprise expression signals (e.g. a promoter, ribosome binding site and/or signal peptide-coding sequence) heterologous to those of Pseudomonas.

In a further aspect, the present invention relates to a replicable expression vector which carries an inserted DNA sequence encoding the Lim factor or a functional analogue thereof, as described above.

In a still further embodiment, the invention relates to a replicable expression vector which carries a first inserted DNA sequence encoding the P. cepacia lipase or a derivative thereof (as described above) and a second DNA sequence encoding the Lim factor (as described above). In this case, the first and second DNA sequences may be expressed from the same promoter although this need not be required. When the first and second DNA sequences are expressed from the same promoter, the lipase-encoding sequence may be located downstream or upstream of the Lim-encoding sequence without adversely affecting the modulation of lipase production effected by the Lim factor.

Alternatively, the DNA sequences encoding the lipase and the Lim-polypeptide, respectively, may each be expressed from a separate promoter, even while carried on the same vector.

In a particular embodiment, the first DNA sequence encoding the lipase and/or the second DNA sequence encoding the Lim factor may further comprise expression signals (e.g. a promoter, ribosome binding site and/or signal peptide-coding sequence) heterologous to those of Pseudomonas.

The replicable expression vector carrying a DNA sequence encoding the lipase and/or Lim factor may be any vector which is capable of replicating autonomously in a given host organism, typically a plasmid or bacteriophage. In the vector, the DNA sequence encoding the lipase and/or Lim factor should be operably connected to a suitable promoter sequence. The promoter may be any DNA sequence which shows transcriptional activity in the host cell and may be derived from genes encoding proteins either homologous or heterologous to the host organism, e.g. a promoter which is heterologous to Pseudomonas. The promoter is preferably derived from a gene encoding a protein homologous to the host organism. Examples of suitable promoters are lac of E.coli, dagA of Streptomyces coelicolor and amyL of Bacillus licheniformis.

The replicable expression vector of the invention further comprises a DNA sequence enabling the vector to replicate in the host cell. Examples of such sequences are the origins of replication of plasmids pUC19, pACYC177, pUB110 and pIJ702.

The vector may also comprise a selectable marker, e.g. a gene whose product confers antibiotic resistance, such as ampicillin, kanamycin, chloramphenicol or tetracyclin resistance, or the dal genes from B.subtilis or B.licheniformis.

The procedures used to ligate the DNA sequences coding for the lipase and/or the Lim factor and the promoter, respectively, and to insert them into suitable vectors containing the information necessary for replication in the host cell, are well known to persons skilled in the art (cf ., for instance, Sambrook et al., op.cit.). It will be understood that the vector may be constructed either by first preparing a DNA construct containing the entire DNA sequence coding for the lipase and for the Lim factor and subsequently inserting this construct into a suitable expression vector, or by preparing separate DNA constructs comprising DNA sequences encoding the lipase or the Lim factor, respectively, or by sequentially inserting DNA fragments containing genetic information for the individual elements (such as the lipase or Lim factor) followed by ligation after each step. It should further be understood that, in this connection, the DNA sequence encoding the lipase may be of genomic origin, while the sequence encoding the Lim factor may be prepared synthetically or vice versa, as described above.

In a still further aspect, the present invention relates to a host cell which contains either two separate DNA constructs comprising a DNA sequence encoding the P. cepacia lipase and a DNA sequence encoding the Lim factor as defined above, respectively, or a DNA construct comprising both DNA sequences on the same fragment; in either case, the DNA constructs may be integrated in the host chromosome which may be an advantage as the DNA sequences are more likely to be stably maintained in the cell. Integration of the DNA constructs into the host chromosome may be performed according to conventional methods, e.g. by homologous recombination. The two DNA sequences may be integrated in the host chromosome in such a way that they are each expressed from a separate promoter. In a particular embodiment, the DNA sequence encoding the lipase and/or the DNA sequence encoding the Lim factor may further comprise expression signals (e.g. a promoter, ribosome binding site and/or signal peptide-coding sequence) heterologous to those of Pseudomonas.

Alternatively, the host cell may be transformed with a replicable expression vector which contains both DNA sequences, or with two vectors containing the DNA sequence encoding the lipase and the DNA sequence encoding the Lim factor, respectively.

As a further alternative, the DNA sequence encoding the lipase may be integrated in the host chromosome, and the DNA sequence encoding the Lim factor may be carried on a replicable expression vector (as described above), or vice versa.

The host cell used in the process of the invention may be any suitable bacterium, yeast or filamentous fungus which, on cultivation, produces large amounts of the P. cepacia lipase.

Examples of suitable bacteria may be grampositive bacteria such as Bacillus subtilis, Bacillus licheniformis, Bacillus lentus, Bacillus brevis, Bacillus stearothermophilus, Bacillus alkalophilus, Bacillus amyloliquefaciens, Bacillus coagulans, Bacillus circulans, or Streptomyces lividans or Streptomyces murinus, or gramnegative bacteria such as E.coli. The transformation of the bacteria may for instance be effected by protoplast transformation or by using competent cells in a manner known per se.

The yeast organism may favourably be selected from a species of Saccharomyces, e.g. Saccharomyces cerevisiae. The filamentous fungus may advantageously belong to a species of Aspergillus, e.g. Aspergillus oryzae or Aspergillus niger. Fungal cells may be transformed by a process involving protoplast formation and transformation of the protoplasts followed by regeneration of the cell wall in a manner known per se. The use of Aspergillus as a host organism is described in, e.g., EP 238 023.

In a yet further aspect, the present invention relates to a method of producing the P. cepacia lipase or a derivative thereof, which method comprises cultivating a host cell as described above under conditions conducive to the production of the lipase or analogue thereof (including conditions ensuring the expression of the Lim factor), and recovering the lipase or analogue from the cells and/or culture medium.

The medium used to cultivate the cells may be any conventional medium suitable for growing bacteria. The lipase may be recovered from the medium by conventional procedures including separating the cells from the medium by centrifugation or filtration, if necessary after disruption of the cells, precipitating the proteinaceous components of the supernatant or filtrate by means of a salt, e.g. ammonium sulphate, followed by purification by a variety of chromatographic procedures, e.g. ion exchange chromatography, affinity chromatography, or the like.

The invention is further illustrated by the following examples which are not in any way intended to limit the scope of the invention, with reference to the appended drawings, wherein
Fig. 1 A-C shows the DNA sequence of the P. cepacia lim gene, the derived amino acid sequence being shown below in the conventional three-letter code,
Fig. 2 A-C shows the DNA sequence encoding the ∼33kD P. cepacia lipase, the derived amino acid sequence being shown below in the conventional three-letter code,
Fig. 3 schematically shows the construction of plasmid pSJ518,
Fig. 4 schematically shows the construction of plasmid pSJ910,
Fig. 5 schematically shows the construction of plasmid pSJ485,
Fig. 6 A-B schematically shows the construction of plasmids pSJ622 and pSJ624,
Fig. 7 schematically shows the construction of plasmid pSJ424,
Fig. 8 schematically shows the construction of plasmids pSJ494 and 909,
Fig. 9 schematically shows the construction of plasmid pSJ729,
Fig. 10 schematically shows the construction of plasmid pSJ416,
Fig. 11 schematically shows the construction of plasmid pSJ600,
Fig. 12 shematically shows the construction of plasmid pSJ671, and
Fig. 13 A-B schematically shows the construction of plasmid pSJ669.

### MATERIALS AND METHODS

### Bacterial strains:

E. coli HW1 is a lacI^{q}, z M15 derivative of strain 803 described in Wood, 1966.

E. coli NM539 is described in Frischauf et al., 1983, and was obtained from Promega.

Pseudomonas cepacia SB10, DSM 3959, is described in WO 89/01032.

Bacillus subtilis DN1885 is a amyE, amyR2, Spo⁺, Pro⁺ derivative of B. subtilis 168.

Bacillus licheniformis ATCC 9789.

Streptomyces lividans TK24 is described in Hopwood et al., 1983.

### Phages:

Lambda EMBL4 is described in Frischauf et al., 1983. It was obtained from Promega.

### Plasmids:

pUC18 and pUC19 are described in Yanisch-Perron et al., 1985.

pACYC177 is described in Chang and Cohen 1978.

pIJ702 is described in Katz et al., 1983.

pIJ4642 is an E.coli cloning vector allowing positive selection for inserts. It resembles pIJ666 (Kieser and Melton, Gene 65, 1988, pp. 83-91) but has more useful sites for cloning. It was obtained from T. Kieser.

pDN1528 is a derivative of the B. subtilis plasmid pUB110 (Gryczan et al.,1978), carrying a 2.3 kb HindIII - SphI fragment containing the alpha-amylase gene from B. licheniformis strain DN52, an amylase-overproducing derivative of ATCC9789 produced by conventional mutagenesis procedures.

pPL1131 is a pUB110-derived plasmid containing the amylase promoter and signal peptide-coding region of pDN1528 followed by a synthetic linker.

pIJ2002 is a pUC18 derivative containing the dagA (agarase) gene of S. coelicolor A3(2). It is described in Buttner et al., 1987, and was obtained from M. Bibb.

### General Methods:

Standard DNA manipulations were performed essentially as described in Maniatis et al., 1982.

Restriction enzymes, T4 DNA ligase, T4 polynucleotide kinase, DNA Polymerase I (Klenow Fragment), and Exonuclease III were obtained from New England Biolabs and used as recommended by the supplier. Nuclease S1 and the restriction enzyme HindII were obtained from Boehringer Mannheim and used as recommended by the supplier.
Chicken egg white lysozyme was obtained from Sigma.

Preparation of plasmids from all strains was conducted by the method described by Kieser (1984).

Lambda DNA was packaged in vitro using the Packagene extract from Promega.

DNA sequencing was performed by the dideoxy chain termination method (Sanger et al., 1977) using ³⁵SdATP (DuPont NEN NEG034H, >1000 Ci/mmol) as the radioactive label and using denatured plasmid DNAs as templates. Sequencing was either carried out as described in Hattori and Sakaki, 1986, using the Klenow fragment, or by means of Sequenase™ as described in the booklet from the supplier (United States Biochemical Corporation). Primers were either the M13 sequencing and reverse sequencing primers from New England Biolabs or oligonucleotides (17-21 mers) complementary to regions of the cloned Pseudomonas DNA prepared by the present inventor.

### Transformation of E. coli:

Cells of E. coli were made competent and transformed as described by Mandel and Higa, 1970.

### Transformation of B. subtilis:

Competent cells were prepared and transformed as described by Yasbin et al., 1975.

### Transformation of B. licheniformis:

Plasmids were introduced into B.licheniformis by polyethylene glycol-mediated protoplast transformation as described by Akamatzu (1984).

### Transformation of S. lividans:

Transformation and other procedures for Streptomyces were as described in Hopwood et al., 1985.

### Oligonucleotide synthesis:

Synthesis was performed on an automatic DNA synthesizer using the phosphoamidite method described by Beaucage and Caruthers, Tetrahedron Letters 22, 1981, pp. 1859-1869. Crude oligonucleotides were purified by polyacrylamide gel electrophoresis.

### Preparation of phage DNA:

Phage lambda DNA was prepared from small-scale liquid cultures as described in Maniatis et al., 1982.

### Exo III deletions:

Unidirectional deletions were made using Exonuclease III as described by Henikoff, 1984.

### Lipase analysis

Lipase was measured by a pH-stat method using tributyrine as substrate. 1 LU (Lipase Unit) is the amount of enzyme which liberates 1 umole titratable butyric acid per minute under the following conditions:

| | |
|---|---|
| Temperature | 30.0 °C |
| pH | 7.0 |
| Emulsifier | Gum Arabic, 1 g/l |
| Substrate | Tributyrine, 50 ml/l |

### Media:

| | | |
|---|---|---|
| TY: | Trypticase | 20 g/l |
| | Yeast extract | 5 g/l |
| | FeCl₂.4H₂O | 6 mg/l |
| | MnCl₂.4H₂O | 1 mg/l |
| | MgSO₄.7H₂O | 15 mg/l |
| | pH | 7.3 |
| BPX: | Potato starch | 100 g/l |
| | Barley flour | 50 g/l |
| | BAN 5000 SKB | 0.1g/l |
| | Sodium caseinate | 10 g/l |
| | Soy Bean Meal | 20 g/l |
| | Na₂HPO₄, 12 H₂O | 9 g/l |
| | Pluronic | 0.1g/l |
| LB agar: | Bacto-tryptone | 10 g/l |
| | Bacto yeast extract | 5 g/l |
| | NaCl | 10 g/l |
| | Bacto agar | 15 g/l |
| Adjusted to pH 7.5 with NaOH | | |

### LB agar with tributyrine:

To 350 ml of melted LB agar is added 3.5 ml tributyrine and 0.35 g Gum Arabic, the mixture is emulsified using an Ultra Turrax emulsifier and then autoclaved.

### Nile Blue indicator plates:

The bottom layer consisted of 15 ml LB agar.
The top layer contained a mixture of 3 ml soft agar (LB with 0.6 % agar), 300 µl olive oil emulsion and 20 µl of a 5% Nile Blue solution (made up in water and sterile filtered).

### Olive Oil emulsion:

| | |
|---|---|
| Olive oil | 10 ml |
| Gum Arabic | 1 g |
| Deionised water | 90 ml |
| mixed using an Ultra Turrax emulsifier. | |

### Buffers:

| | |
|---|---|
| TE buffer: | 10 mM Tris, pH 8, 1 mM EDTA, pH 8. |
| Phage buffer: | 0.01 M NaCl |
| | 0.01 M MgCl₂ |
| | 0.01 M Tris.HCl, pH 7.0 |
| Ligation buffer: | 0.066 M Tris.HCl pH 7.5, 0.01 M MgCl₂, 25 µg/ml gelatine, 0.001 M ATP, 0.01 M DTT. |

### EXAMPLE 1

### Cloning of the Pseudomonas cepacia lipase gene

### A) Preparation of chromosomal DNA from Pseudomonas cepacia SB10.

A pellet of frozen cells from a 3 ml TY culture was resuspended in 1 ml TE buffer containing 1 mg/ml lysozyme. 0.1 ml EDTA (0.5 M, pH 8.0) was added and the mixture was incubated at 37 °C for 15 min. with gentle shaking. 5 µl 20% SDS was added, and the solution was repeatedly extracted with phenol (300 µl portions) until no interface was present. The supernatant was then extracted once with CHCl₃ (300 µl). To 900 µl supernatant, 90 µl 3 M Na-acetate and 500 µl isopropanol were added. After 5-10 minutes, the precipitated DNA was recovered on a plastic inoculating loop, washed in 70 and 96 % EtOH and resuspended in 500 µl TE. The solution of chromosomal DNA (chrDNA) was kept at -20 °C.

### B) Partial cutting and fractionation of chrDNA from P. cepacia.

80 µg chrDNA from P.cepacia SB10 (prepared as described in section A above) was digested with 5 units Sau3A at 37 °C for 5 minutes in 400 µl of the buffer recommended by the supplier, and heated at 70 °C for 10 min, and the total sample was then loaded on a preparative agarose gel. After electrophoresis, gel segments containing DNA fragments of 9-23 kb were cut out. The chromosomal DNA fragments were recovered in the liquid formed after freezing/thawing the gel segments, extracted twice with phenol, once with CHCl₃, ethanol precipitated and dissolved in TE buffer.

### C) Construction of a P. cepacia DNA library in phage lambda.

2 µg lambda EMBL4 DNA was digested completely with restriction enzymes BamHI and SalI, extracted twice with phenol, once with CHCl₃, ethanol precipitated and resuspended in 10 µl TE. The suspension was mixed with 2.5 µg size-fractionated chrDNA (in 50 µl TE), ethanol precipitated, resuspended in 10 µl ligation buffer, 100 units of T4 DNA ligase were added and ligation was carried out for 2 h at room temperature followed by 16 hours at 4°C. The ligated DNA was then packaged into lambda phage particles using the Packagene™ extract from Promega, diluted in 500 µl phage buffer and 50 µl CHCl₃ were added. This was used for making an amplified phage stock as follows: Packaged phage particles were mixed with an equal volume of a fresh overnight culture of E. coli NM539 grown in TY + 0.4 % maltose + 20 mM MgSO₄, allowed to adsorb for 20 minutes at 37°C and plated on fresh LB plates in 4 ml soft LB agar (0.6 %) containing 20 mM MgSO₄. After incubation at 37°C for 16 hours, the partially lysed top layer containing about 2500 plaques was scraped into a 40 ml centrifuge tube containing 5 ml phage buffer, and phage eluted for 2 hours. The supernatant after centrifugation at 5000 rpm for 5 min. in a Sorvall SS34 rotor was kept as an amplified stock and used for screening.

### D) Isolation of a recombinant lambda phage carrying a lipase gene.

About 200000 phages from the amplified library were adsorbed to NM539 and plated on 20 LB plates in soft agar containing 1 % glycerol tributyrate emulsion, resulting in total lysis on each plate. 170 clear plaques (hydrolysing the tributyrine) were identified in the turbid tributyrine emulsion, and a number reisolated. 35 plaques were streaked on Nile Blue indicator plates and 10 of these gave a positive reaction, i.e. a strong blue colouring. Phage DNA was isolated from three of these phages, which were found to be identical, based on restriction enzyme digests.

### E) Subcloning into pUC19

1 µg of the phage DNA prepared in section D above was partially cut with SalI, mixed with 0.3 µg SalI digested pUC19 DNA, ligated, transformed into competent E. coli HW1 and plated on LB plates containing 200 µg/ml ampicillin. These plates were replicated onto LB plates with 200 µg/ml ampicillin and 1 % glycerol tributyrate emulsion, and colonies surrounded by clear halos were isolated. One of these, SJ150, was shown to harbour a plasmid, designated pSJ150, of approximately 6 kb, including the lipase-coding gene and the lipase modulating (lim) gene.

### F) DNA sequencing

The sequence of most of the cloned DNA contained on pSJ150 was determined by the dideoxy chain terminating method directly on double-stranded templates, as described in Materials and Methods above. The sequences of both strands were determined, using a combination of subcloning of restriction fragments, deletions from either end of the cloned DNA using Exonuclease III, and synthetic oligonucleotide primers. This allowed identification of the lipase coding sequence shown in Fig. 2A-C, and the lipase modulator (lim) coding sequence shown in Fig. 1A-C. On pSJ150, the sequence TCG separates the lipase modulator start codon (ATG) from the lipase stop codon (TAA).

### EXAMPLE 2

### Expression of the Pseudomonas cepacia lipase

### A. Expression of the unmodified lipase in E.coli

### (1) lip without lim

Plasmid pSJ518 was constructed by subcloning the 1.5 kb HindIII-ClaI fragment containing the lipase (lip) gene from pSJ150 into pUC19, as shown in Fig. 3. Strain SJ518 (E. coli HW1 containing pSJ518) does not produce any lipase as seen on plates containing 1 % glycerol tributyrate (i.e. no halos surround the colonies).

### (2) lip + lim on the same plasmid

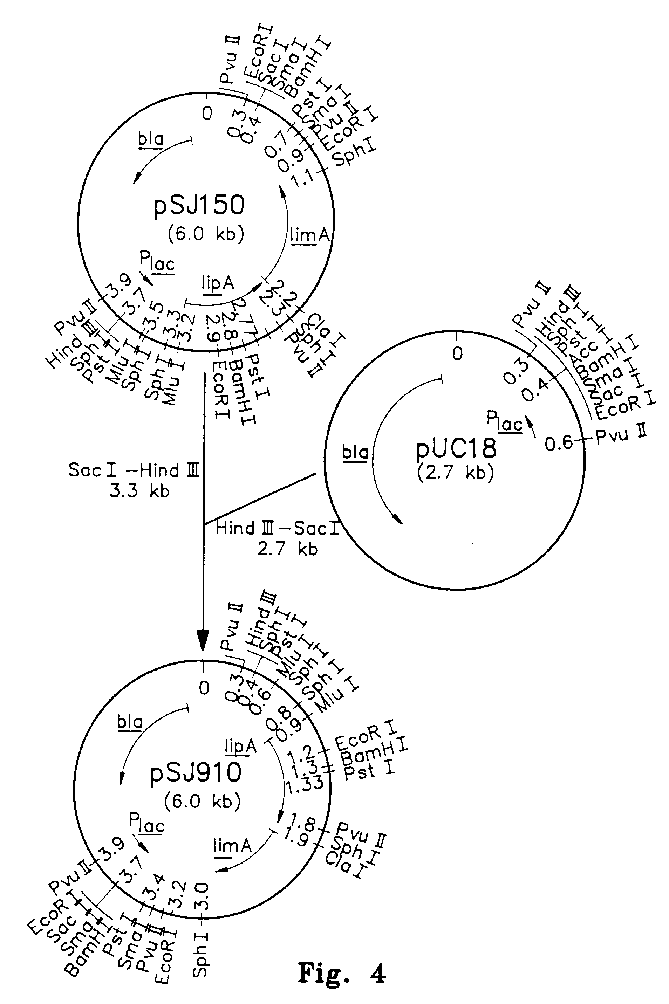

On pSJ150, the lipase is expressed from its own as well as from the pUC19 lac promoter. The plasmid pSJ910, containing the same fragment of cloned DNA but in the reverse orientation with respect to the lac promoter, was constructed as outlined in Fig. 4 and introduced into E. coli HW1 to form strain SJ910. When plated on plates containing 1 % glycerol tributyrate, SJ910 colonies were surrounded by smaller halos than colonies of SJ150. This indicates the presence of a promoter, active in E. coli, for the lip and lim genes.

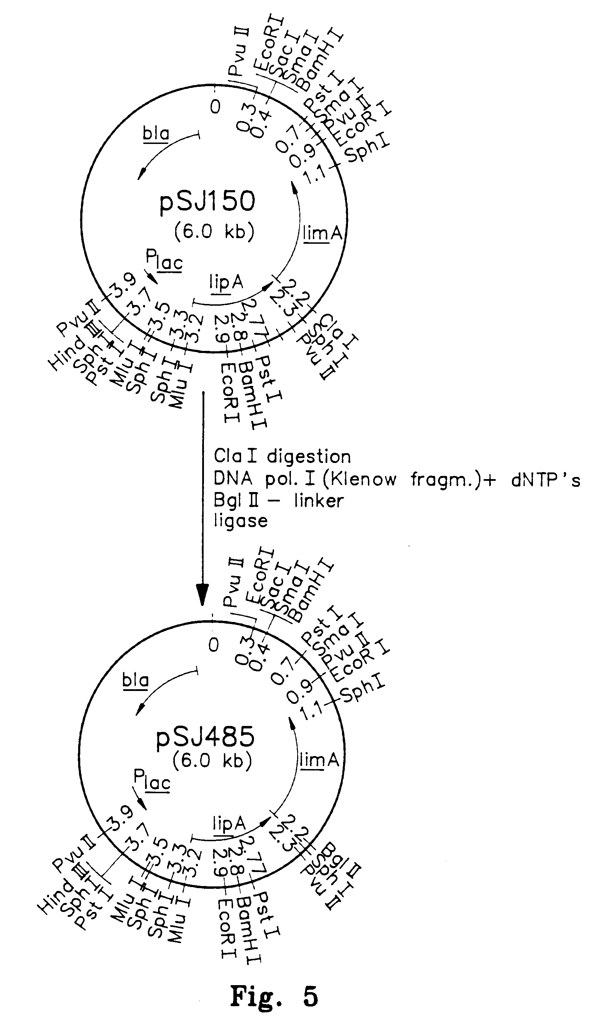

The region between the lip and lim genes can be changed without negatively affecting lipase expression. pSJ150 contains a unique ClaI site one basepair after the lipase stop codon. Plasmid pSJ485 was constructed by insertion of an 8 basepair BglII linker (New England Biolabs) into this ClaI site which had been filled-in using the large fragment of DNA polymerase I (Klenow fragment) and dNTP's, thus giving a 10 basepair insertion (Fig. 5). Strain SJ485 (E. coli HW1 containing pSJ485) produced the same amounts of lipase as SJ150.

### (3) lip and lim on separate plasmids

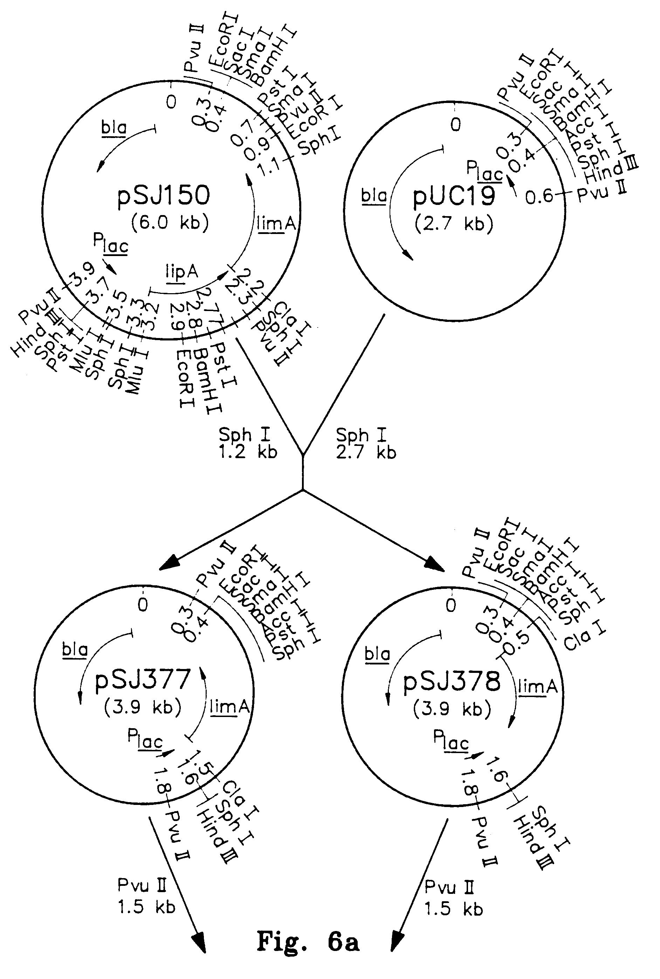

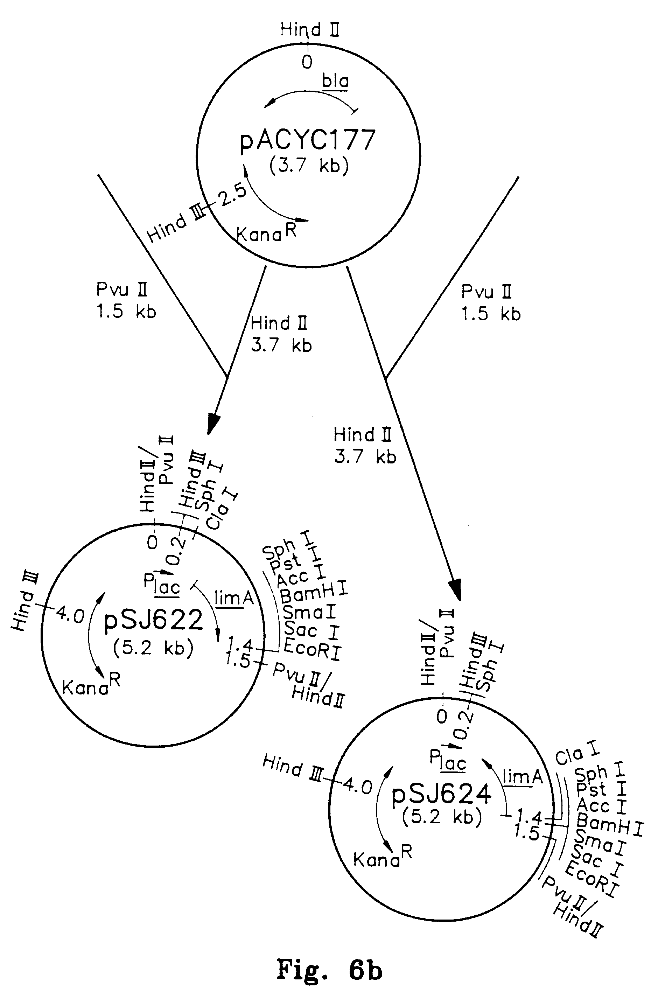

The lim gene was excised on a 1.2 kb SphI fragment from pSJ150 and inserted into the SphI site of pUC19. This resulted in plasmids pSJ377, with the lac promoter reading correctly into the lim gene, and pSJ378, with the lac promoter reading backwards into the lim gene. From pSJ377 and pSJ378, the 1.2 kb insert + the lac promoter could be excised as a 1.5 kb PvuII fragment. This was inserted into the HindII site of pACYC177 to give pSJ622 (insert from pSJ377) and pSJ624 (insert from pSJ378). The construction of pSJ622 and pSJ624 is outlined in Fig. 6.

Plasmids pSJ622 and pSJ624 were introduced into competent SJ518 (the strain containing pSJ518 with the lipase gene alone), selecting for ampicillin and kanamycin resistance, and the transformants were replicated onto plates containing glycerol tributyrate. Large halos were formed around transformants containing pSJ622, but none around transformants containing pSJ624, indicating lipase production from the former but not from the latter.

### B. Expression of a modified lipase gene in E.coli

### (1) lip without lim

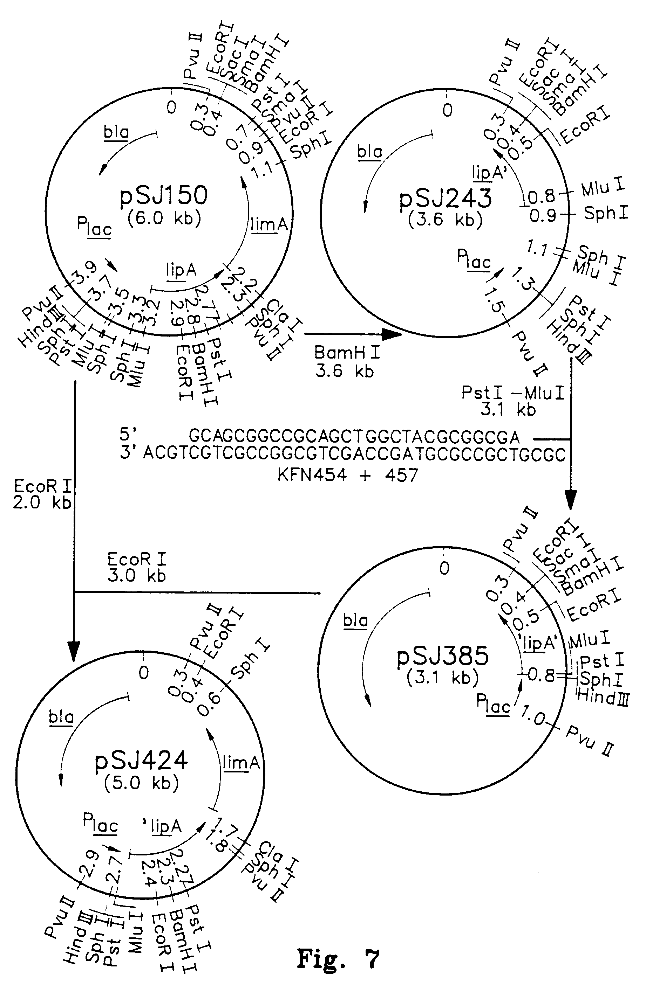

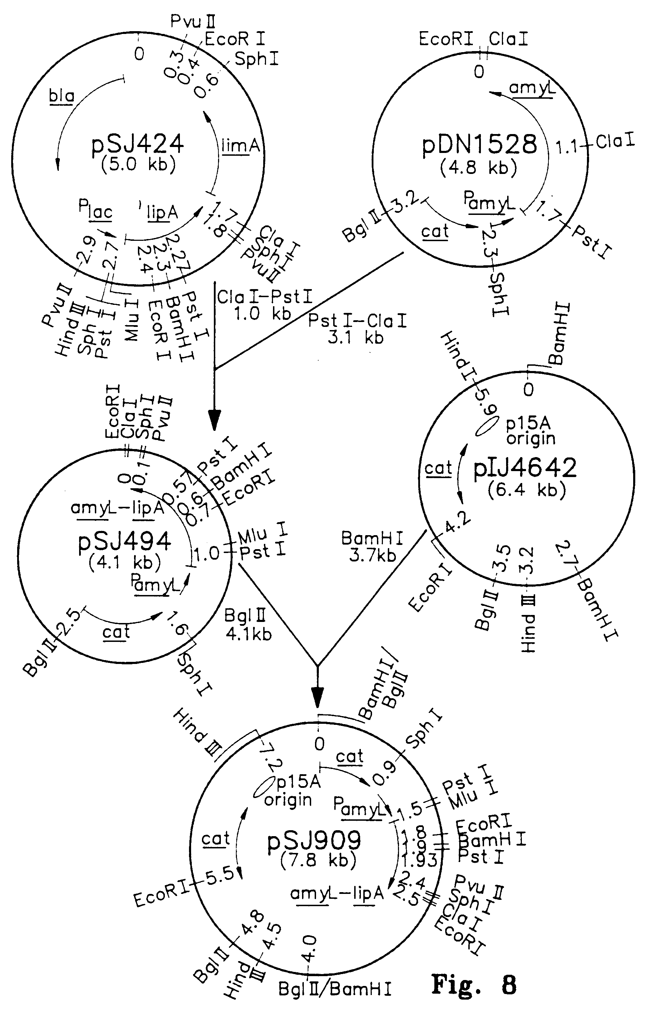

pSJ494 is an expression plasmid for the P.cepacia lipase in Bacillus. It has the promoter, ribosome binding site and signal peptide coding region from the B. licheniformis alpha-amylase gene fused in-frame to DNA coding for the mature lipase, but does not contain the lim gene. The construction of pSJ494 is outlined in Figs. 7 and 8. The sequence of the fusion region is as follows: 
ATG AAA CAA CAA AAA CGG CTT TAC GCC CGA TTG CTG ACG CTG
Met Lys Gln Gln Lys Arg Leu Tyr Ala Arg Leu Leu Thr Leu
alpha-amylase signal peptide
 
PstI
TTA TTT GCG CTC ATC TTC TTG CTG CCT CAT TCT GCA GCA GCG GCC
Leu Phe Ala Leu Ile Phe Leu Leu Pro His Ser Ala Ala Ala Ala

MluI
GCA GCT GGC TAC GCG GCG ACG CGT TAC CCG ATC - -
Ala Ala Gly Tyr Ala Ala Thr Arg Tyr Pro Ile - -
¦ mature lipase sequence
In order to introduce this alpha-amylase/lipase fusion gene into E. coli, plasmid pSJ909 was constructed (Fig. 8). Strain SJ909 (E. coli HW1 containing pSJ909) does not produce any halos on plates containing glycerol tributyrate.

### (2) lip and lim on separate plasmids

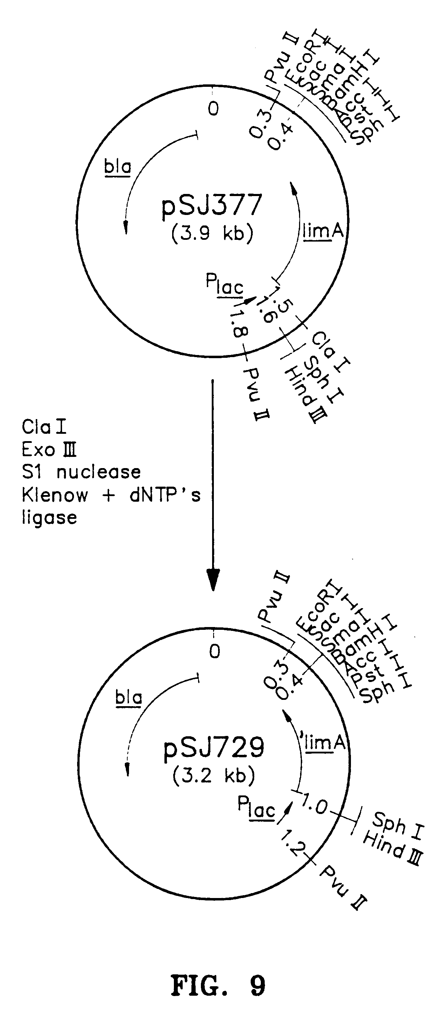

Strain SJ909 was made competent and transformed with either pSJ377 (constructed as shown in Fig. 6) or pSJ729, which was derived from pSJ377 by deleting about 600 basepairs from the 5' terminal of the lim gene (Fig. 9). When the double transformants were streaked on plates containing glycerol tributyrate, those containing pSJ377 (the lim⁺ plasmid) formed clear halos around the colonies, indicating lipase production, whereas those containing pSJ729 (lim⁻⁾ did not form any halos.

### C. Expression of a modified lipase gene in B.subtilis

### (1) lip without lim

Plasmids pSJ493 and pSJ495 are identical to pSJ494, described in section B.1), carrying the amylase-lipase fusion but no lim gene. They were introduced into B. subtilis strain DN1885 and transformants were grown for 4 days at 37 °C in shake flasks containing BPX growth medium, with the addition of oleyl alcohol to 30 g/l and chloramphenicol to 12 µg/ml.

_{L}ipase activity in the culture broth was measured by the LU-titrimetric method (described in Materials and Methods) and was 1-2 LU/ml. This is not above the background level found for strain DN1885, which varies between 0-5 LU/ml.

### (2) lip and lim on the same plasmid

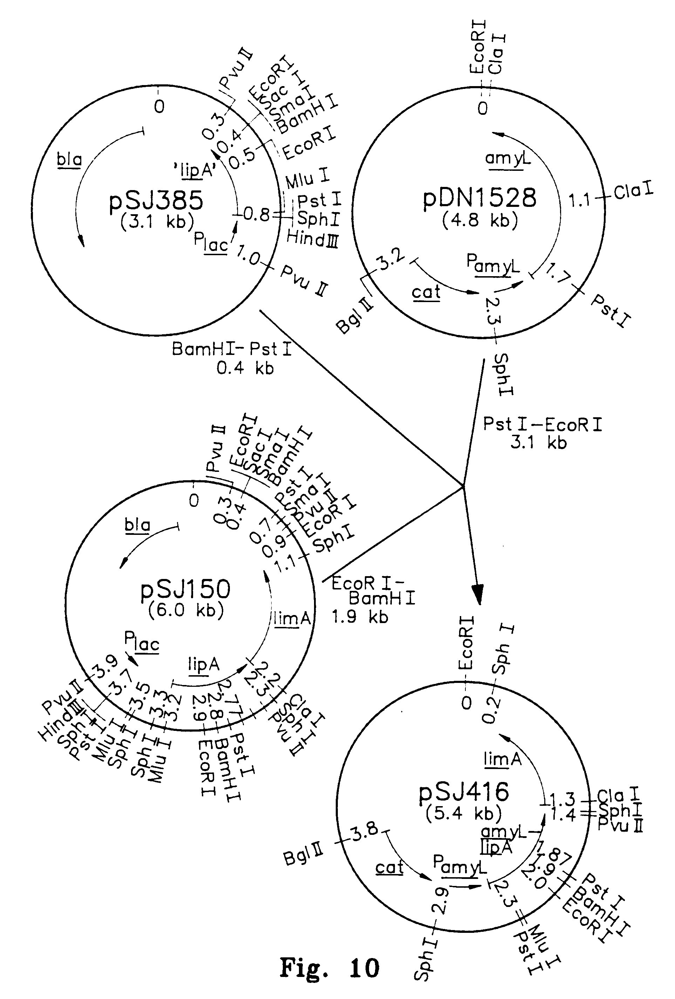

Plasmid pSJ416 contains the same amylase-lipase fusion as pSJ493-495, but the lipase gene is followed immediately down-stream by the lim gene in exactly the same way as on pSJ150 (the construction of pSJ416 is outlined in Fig. 10).

When this plasmid was introduced into B. subtilis DN1885 and transformants grown in shake flasks as described above, yields reached 40 LU/ml in 4 days.

### D. Expression of a modified lipase gene in B.licheniformis

### (1) lip without lim

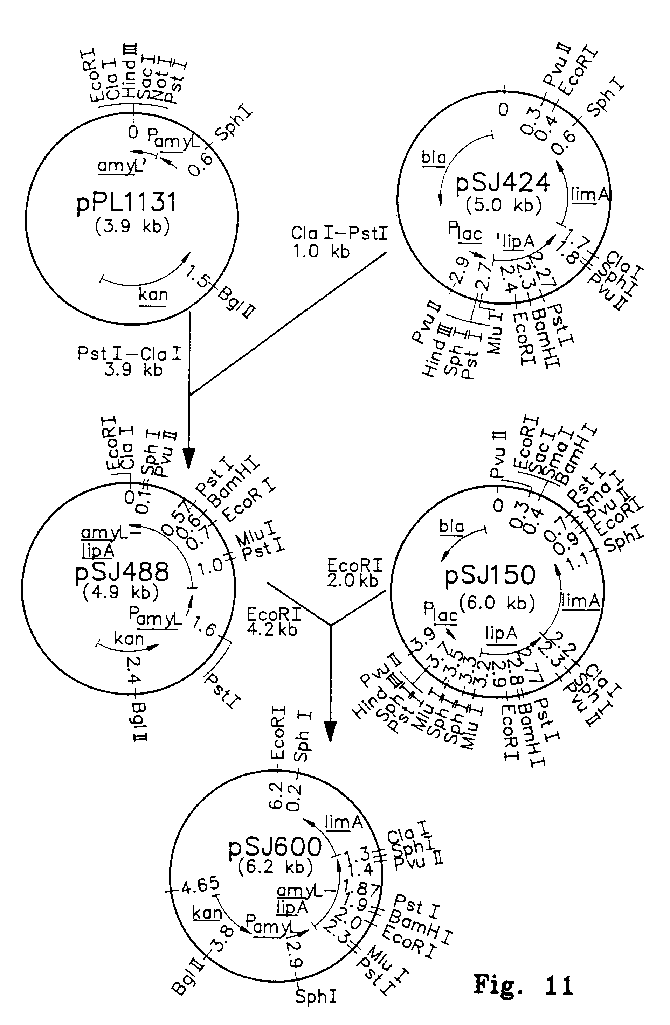

Plasmid pSJ488 contains the same amylase-lipase fusion as pSJ493, but on the Bacillus vector pPL1131 conferring kanamycin resistance to its host strain (Fig. 11). This plasmid was introduced into B. licheniformis strain ATCC 9789 by protoplast transformation, and the transformants were streaked on plates containing glycerol tributyrate. The halos formed around the transformants were very small, of the same size as those formed around the untransformed strain.

### (2) lip and lim on the same plasmid

Plasmid pSJ600 contains the same amylase-lipase fusion as pSJ488, but the lipase gene is followed immediately downstream by the lim gene in exactly the same way as on pSJ150 (the construction of pSJ600 is outlined in Fig. 11). When this plasmid was introduced into B. licheniformis ATCC 9789 by protoplast transformation, and the transformants were streaked on plates containing glycerol tributyrate, pronounced halos were formed around the colonies indicating lipase production.

### E. Expression of a modified lipase gene in S.lividans

### (1) lip without lim-expression

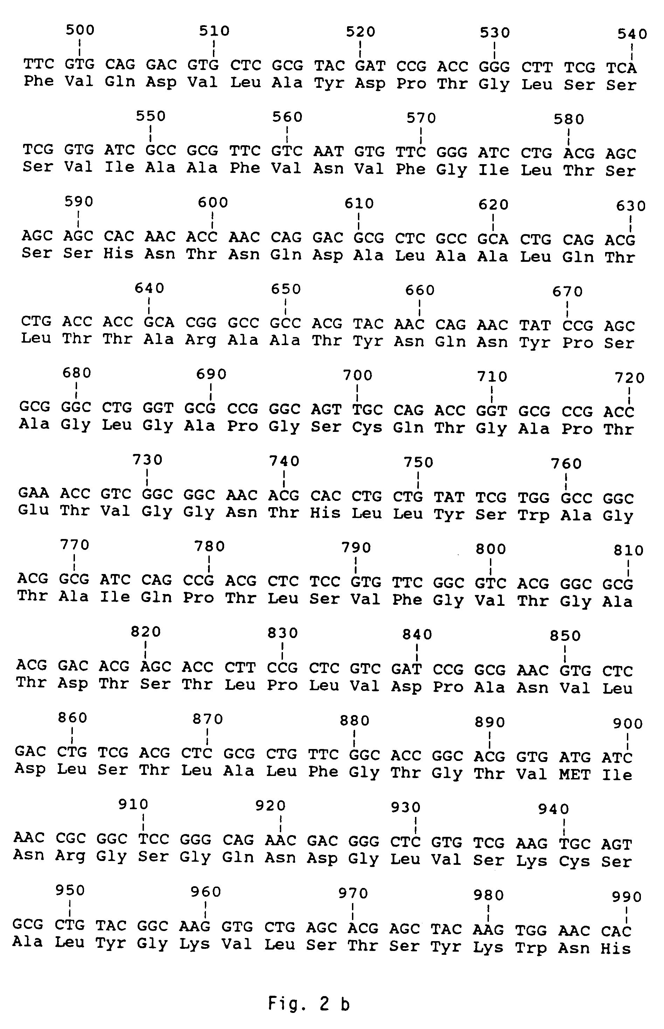

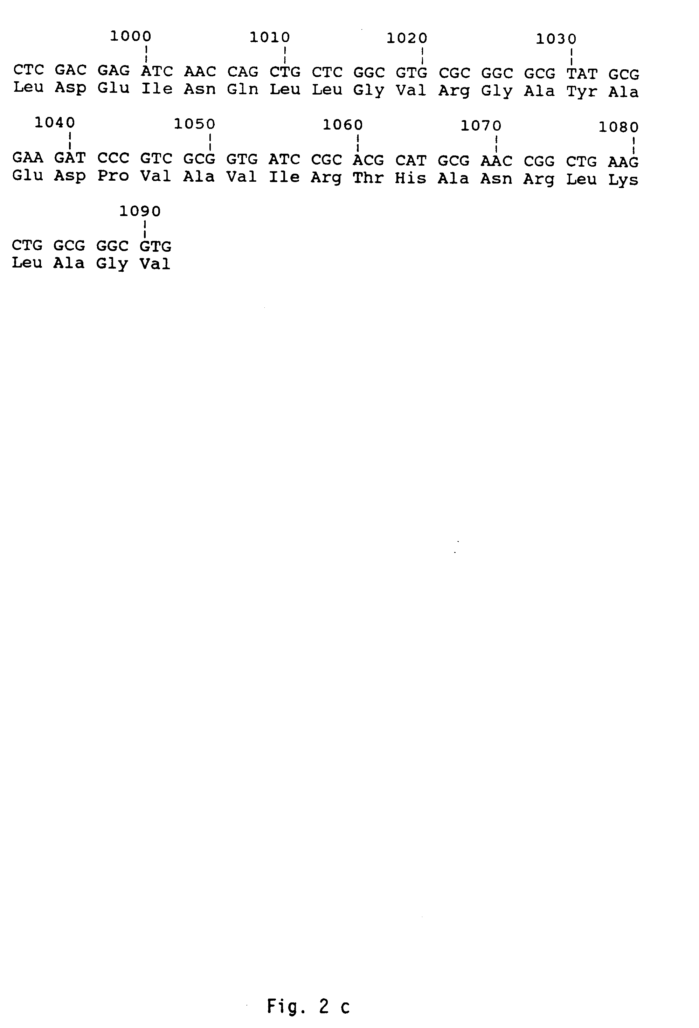

Plasmid pSJ604 was constructed by isolating a 880 bp HindIII-AvaII fragment from plasmid pIJ2002, carrying the dagA promoters and part of the signal peptide-encoding sequence (cf. Fig. 2 in Buttner et al., 1987). To the fragment were added two complementary, phosphorylated and annealed oligonucleotides of the following sequence: 
AvaII MluI XhoI

GTCCCGCACCCGCCGCTCATGCCGCAGCTGGCTACGCGGCGACGCGTC

GGCGTGGGCGGCGAGTACGGCGTCGACCGATGCGCCGCTGCGCAGAGCT
This mixture was ligated to pUC19 which had previously been digested with HindIII and SalI to give plasmid pSJ604.

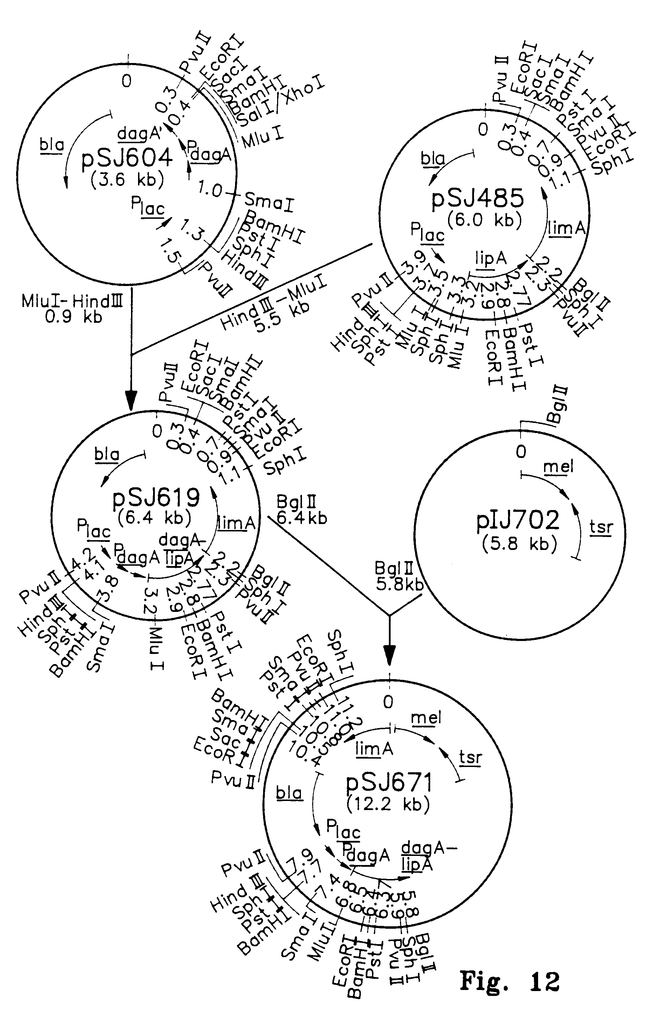

Plasmid pSJ671 is an E. coli-S. lividans shuttle plasmid carrying an in-frame fusion between the promoter, ribosome binding site and signal peptide coding region of the S. coelicolor agarase gene (dagA, Buttner et al., 1987) and the DNA coding for the mature lipase. The lim gene is present on the plasmid, but in such a position that it is not transcribed by any known promoters. The construction of pSJ671 is outlined in Fig. 12, and the sequence of the fusion region is as follows: 
GTG GTC AAC CGA CGT GAT CTC ATC AAG TGG AGT GCC GTC GCA
Met Val Asn Arg Arg Asp Leu Ile Lys Trp Ser Ala Val Ala
agarase signal peptide
 
AvaII
CTC GGA GCG GGT GCG GGG CTC GCG GGT CCC GCA CCC GCC GCT CAT
Leu Gly Ala Gly Ala Gly Leu Ala Gly Pro Ala Pro Ala Ala His

MluI
GCC GCA GCT GGC TAC GCG GCG ACG CGT TAC CCG ATC -
Ala Ala Ala Gly Tyr Ala Ala Thr Arg Tyr Pro Ile
¦ mature lipA sequence
When pSJ671 was introduced into S. lividans TK24 by protoplast transformation and the resulting transformants were streaked on plates containing glycerol tributyrate, very small halos were formed around the colonies, indicating little or no lipase production.

### (2) lip and lim on the same plasmid

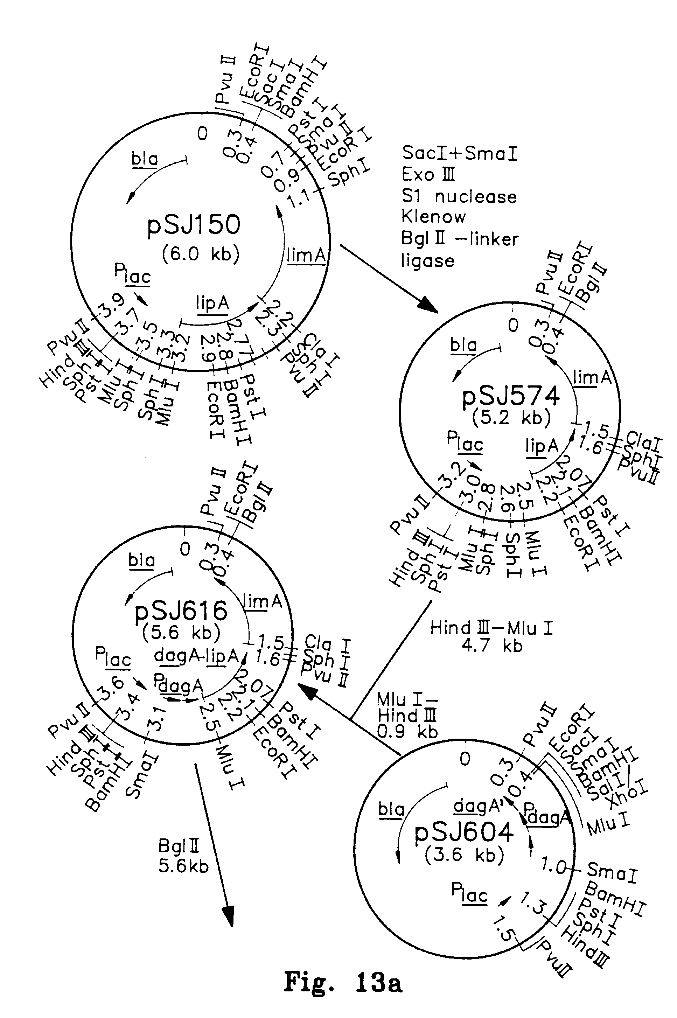

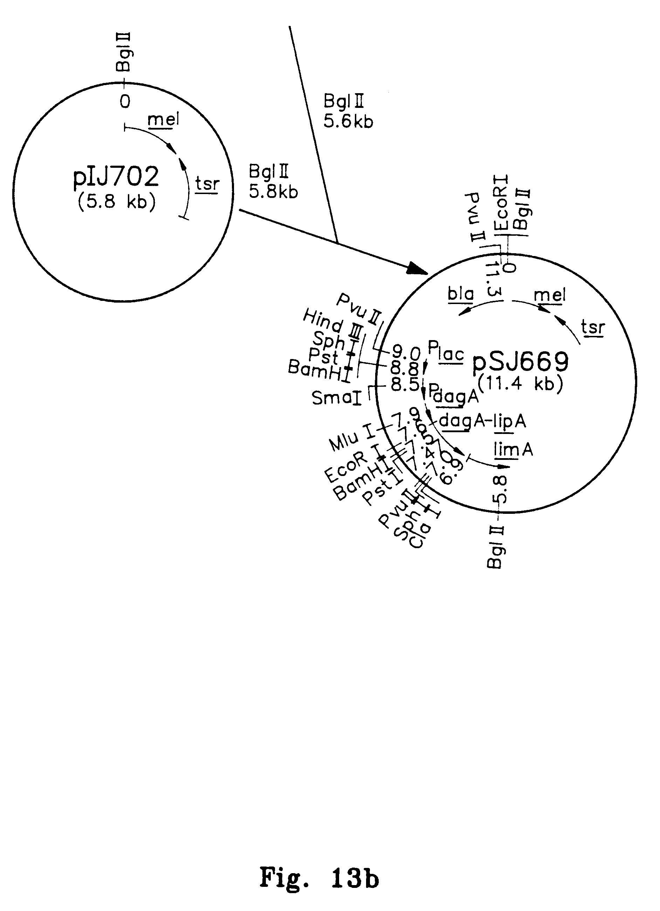

pSJ669 is similar to pSJ671 with the exception that pSJ669 contains the lim gene immediately downstream from the lipase gene in exactly the same way as on pSJ150 (the construction of pSJ669 is outlined in Fig. 13).

When pSJ669 was introduced as described above into S. lividans TK24 and transformants were streaked on plates with glycerol tributyrate, very large halos were formed around the colonies, compared to those around pSJ671.

### F. Effect of a modified lim gene

pSJ377, shown in Fig. 6, contains the lim gene expressed from a lacZ promoter. This plasmid was digested with ClaI, and deletions around the ClaI site were generated using ExoIII. One such deletion plasmid is pSJ721, in which 15 base pairs were deleted, which created a fusion protein derived from the 9 N-terminal codons from the pUC19 lacZ gene plus 8 C-terminal codons from the lip gene fused to a lim gene lacking only the first two codons. The sequence of the fusion region is as follows: 
LacZ start                    SphI     lip C-terminal part
ATG ACC ATG ATT ACG CCA AGC TTG CAT GCG AAC CGG CTG AAG CTG
Met Thr Met Ile Thr Pro Ser Leu His Ala Asn Arg Leu Lys Leu

   : lim from 3. codon
GCG GGG:GCA CGA GGA GGA CGC GCG CCG CTG GCG CGC CGC GCC GTG
Ala Gly Ala Arg Gly Gly Arg Ala Pro Leu Ala Arg Arg Ala Val

GTC
Val
The fusion construct plus the lac promoter was excised from pSJ721 as a 1.5 kb PvuII fragment and inserted into the HindII site of pACYC177 to give pSJ812, in the same manner as in the construction of pSJ622 shown in Fig. 6.

pSJ812 was introduced into competent SJ518 (the strain containing pSJ518 with the lipase gene alone), selecting for ampicillin and kanamycin resistance, and the transformants were replicated onto plates containing glycerol tributyrate. The halos formed around the transformants were of approximately the same size as those formed around transformants of SJ518 with pSJ622, expressing the non-modified lim gene.

### EXAMPLE 3

### Expression and identification of the lim encoded polypeptide

The lim gene was excised from pSJ150 as a 1.17 kb ClaI - SphI fragment and inserted into the NdeI site of plasmid pJW2 (Wang et al., 1990) after creating blunt ends with the Klenow fragment of DNA polymerase I. Recombinant plasmids were obtained with the lim gene inserted in either of the two possible orientations. pCBE3 and pCBE4 both contain the lim gene in the correct orientation for transcription from the temperature-inducible lambda pR and pL promoters on pJW2, whereas pCBE2 contain the lim gene in the opposite orientation. The plasmids were introduced into E. coli JA221 (Clarke and Carbon, 1978), and transcription from the pL and pR promoters was induced by increasing the temperature of the cultures to 42 °C. Proteins produced upon induction were identified by polyacrylamide gel electrophoresis of cells harvested from the induced cultures. This allowed identification of a protein of an apparent molecular weight of 34000 which was found in induced cultures of transformants carrying pCBE3 and pCBE4, but not in the induced culture of the transformant carrying pCBE2, or in uninduced cultures.

### REFERENCES.

Akamatzu, T., Sekiguchi, J. (1984). An improved method of protoplast regeneration for Bacillus species and its application to protoplast fusion and transformation. Agric. Biol. Chem. 48, 651-655.

Buttner, M. J., Fearnley, I. M., Bibb, M. J. (1987). The agarase gene (dagA) of Streptomyces coelicolor A3(2): Nucleotide sequence and transcriptional analysis. Mol. Gen. Genet. 209, 101-109.

Chang, A. C. Y., Cohen, S. N. (1978). Construction and Amplification of Amplifiable Multicopy DNA Cloning Vehicles Derived from the p15A Cryptic Miniplasmid. J. Bacteriol. 134, 1141-1156.

Clarke, L. and Carben, J. (1978). J.Mol.Biol. 120, pp. 517-534.

Frischauf, A.-M., Lehrach, H., Poutstka, A., Murray, N. (1983). Lambda Replacement Vectors Carrying Polylinker Sequences. J. Mol. Biol. 170, 827-842.

Hattori, M., Sakaki, Y. (1986). Dideoxy Sequencing Method Using Denatured Plasmid Templates. Anal. Biochem. 152, 232-238.

Henikoff, S. (1984). Unidirectional digestion with exonuclease III creates targeted breakpoints for DNA sequencing. Gene 28, 351-359.

Hopwood, D. A., Bibb, M. J., Chater, K. F., Kieser, T., Bruton, C. J., Kieser, H. M., Lydiate, D. J., Smith, C. P., Ward, J. M., Schrempf, H. (1985). Genetic manipulation of Streptomyces. A laboratory manual. The John Innes Foundation, Norwich.

Hopwood, D. A., Kieser, T., Wright, H. M., Bibb, M. J. (1983). Plasmids, Recombination and Chromosome Mapping in Streptomyces lividans 66. J. Gen. Microbiol. 129, 2257-2269.

Katz, E., Thompsom, C. J., Hopwood, D. A. (1983). Cloning and expression the tyrosinase gene from Streptomyces antibioticus in Streptomyces lividans. J. Gen. Microbiol. 129, 2703-2714.

Kieser, T. (1984). Factors affecting the isolation of CCC DNA from Streptomyces lividans and Escherichia coli. Plasmid 12, 19-36.

Mandel, M., Higa, A. (1970). Calcium-dependent bacteriophage DNA infection. J. Mol. Biol. 53, 159-162.

Maniatis, T., Fritsch, E. F., Sambrook, J. (1982). Molecular Cloning: A Laboratory Manual. Cold Spring Harbor Laboratory, Cold Spring Harbor, New York.

Yanisch-Perron, C., Vieira, J., Messing, J. (1985). Improved M13 phage cloning vectors and host strains: Nucleotide sequences of the M13mp18 and pUC19 vectors. Gene, 33, 103-119.

Yasbin, R. E., Wilson, G. A., Young, F. E. (1975) Transformation and transfection of lysogenic strains of Bacillus subtilis: evidence for selective induction of prophage in competent cells. J. Bacteriol. 121, 296-304.

Wang, H. et al. (1990). An efficient temperature-inducible vector incorporating the T7 gene 10 translation initiation region. Nucl. Acids Res. 18, p. 1070.

Wood, W. B. (1966). Host specificity of DNA produced by Escherichia coli: Bacterial mutations affecting the restriction and modification of DNA. J. Mol. Biol. 16, 118-133.

## Claims

1. A DNA construct which comprises a DNA sequence as shown in the appended Fig. 1A-C, encoding a factor which acts in trans as a modulator of the production of a Pseudomonas cepacia lipase, or a modification of said DNA sequence encoding a functional analogue of the P. cepacia lipase modulating factor, the P. cepacia lipase modulating factor or functional analogue thereof being capable of modulating lipase production when the P. cepacia lipase is expressed using expression signals which are heterologous to Pseudomonas and/or when the P. cepacia lipase is expressed in a heterologous host cell.

2. A DNA construct according to Claim 1, wherein the DNA sequence is derived from the chromosome of P. cepacia.

3. A DNA construct according to Claim 2, wherein the DNA sequence is located on the P. cepacia chromosome downstream of a DNA sequence encoding a ∼33 kD lipase.

4. A DNA construct which comprises a first DNA sequence encoding a P. cepacia lipase or a derivative thereof, and a second DNA sequence encoding a P. cepacia lipase modulating factor as defined in any of Claims 1 - 3.

5. A DNA construct according to Claim 4, wherein the first DNA sequence encodes a ∼33 kD lipase.

6. A DNA construct according to Claim 4, wherein the DNA sequence is the one shown in the appended Fig. 2 A-C or a modification thereof encoding a derivative of the P. cepacia lipase.

7. A DNA construct according to Claim 4, wherein the DNA sequence encoding the P. cepacia lipase is located upstream of the DNA sequence encoding P. cepacia lipase modulating factor.

8. A DNA construct according to Claim 4, wherein the DNA sequence encoding the P. cepacia lipase is located downstream of the DNA sequence encoding the P. cepacia lipase modulating factor.

9. A DNA construct according to any of Claims 4 - 8, wherein the first and/or second DNA sequence further comprises expression signals heterologous to those of Pseudomonas.

10. A replicable expression vector which carries an inserted DNA sequence as shown in the appended Fig. 1A-C, encoding a factor which acts in trans as a modulator of the production of a P. cepacia lipase, or a modification of said DNA sequence encoding a functional analogue of the P. cepacia lipase modulating factor, the P. cepacia lipase modulating factor or functional analogue thereof being capable of modulating lipase production when the P. cepacia lipase is expressed using expression signals which are heterologous to Pseudomonas and/or when the P. cepacia lipase is expressed in a heterologous host cell.

11. A vector according to Claim 10, wherein the DNA sequence is as defined in Claim 2 or 3.

12. A replicable expression vector which carries a first inserted DNA sequence encoding a P. cepacia lipase or a derivative thereof, and a second inserted DNA sequence encoding a P. cepacia lipase modulating factor as defined in any of Claims 1 - 3.

13. A vector according to Claim 12, wherein the first DNA sequence encodes a ∼33 kD lipase.

14. A vector according to Claim 13, wherein the DNA sequence is the one shown in the appended Fig. 2 A-B or a modification thereof encoding a derivative of the P. cepacia lipase.

15. A vector according to Claim 12, wherein the first and second DNA sequence are expressed from the same promoter.

16. A vector according to Claim 15, wherein the first DNA sequence encoding the P. cepacia lipase is located upstream of the second DNA sequence encoding the P. cepacia lipase modulating factor.

17. A vector according to Claim 15, wherein the first DNA sequence encoding the P. cepacia lipase is located downstream of the second DNA sequence encoding the P. cepacia lipase modulating factor.

18. A vector according to Claim 12, wherein the first DNA sequence encoding the P.cepacia lipase and the second DNA sequence encoding the P. cepacia lipase modulating factor are each expressed from a separate promoter.

19. A vector according to any of Claims 12 - 18, wherein the first and/or second DNA sequence further comprises expression signals heterologous to those of Pseudomonas.

20. A host cell containing a first DNA construct comprising a DNA sequence encoding a P. cepacia lipase or a derivative thereof, and a second DNA construct according to any of Claims 1 - 3.

21. A host cell according to Claim 20, wherein the first DNA construct encodes a ∼33 kD lipase.

22. A host cell according to Claim 21, wherein the DNA sequence of the DNA construct is the one shown in the appended Fig. 2 A-B or a modification thereof encoding a derivative of the P. cepacia lipase.

23. A host cell according to any of Claims 20 - 22, wherein the first and/or second DNA construct further comprises expression signals heterologous to those of Pseudomonas.

24. A host cell containing a DNA construct according to any of Claims 4 - 9.

25. A host cell according to Claim 20, which is transformed with a replicable expression vector carrying the first DNA construct, and with a replicable expression vector according to Claim 10.

26. A host cell according to Claim 25, wherein the DNA sequence encoding the P. cepacia lipase further comprises expression signals heterologous to those of Pseudomonas.

27. A host cell according to Claim 20 which is transformed with a replicable expression vector according to any of Claims 12 - 19.

28. A host cell according to Claim 20, which carries the first DNA construct integrated in its chromosome, and which contains a replicable expression vector according to Claim 10.

29. A host cell according to Claim 20, which carries the second DNA construct integrated in its chromosome, and which contains a replicable expression vector containing the first DNA construct.

30. A host cell according to Claim 20, which carries the first and second DNA construct integrated in its chromosome in such a way that the DNA sequence coding for the P. cepacia lipase and the DNA sequence coding for the lipase modulating factor are each expressed from a separate promoter.

31. A host cell according to any of Claims 22 - 30 which is a bacterium.

32. A host cell according to Claim 31 which is a grampositive bacterium.

33. A host cell according to Claim 32, wherein the grampositive bacterium is selected from the group consisting of Bacillus subtilis, Bacillus licheniformis, or Streptomyces lividans.

34. A host cell according to Claim 31 which is a gramnegative bacterium.

35. A method of producing a P. cepacia lipase or a derivative thereof, which method comprises cultivating a host cell according to any of Claims 20 - 34 under conditions conducive to the production of the lipase or analogue, and recovering the lipase or analogue from the culture medium.

## Patentansprüche

1. DNA-Konstrukt, das eine DNA-Sequenz, die in den beigefügten Figg. 1 A-C gezeigt ist und für einen Faktor kodiert, der in trans als Modulator der Bildung einer Pseudomonas cepacia-Lipase wirkt, oder eine Modifizierung der DNA-Sequenz, die für ein funktionelles Analogon des P. cepacia-Lipase-Modulierungsfaktors kodiert, unfaßt, wobei der P. cepacia-Lipase-Modulierungsfaktor oder das funktionelle Analogon davon imstande sind, die Lipasebildung zu modulieren, wenn P. cepacia-Lipase unter Verwendung von Expressionssignalen exprimiert wird, die heterolog gegenüber Pseudomonas sind, und/oder wenn P. cepacia-Lipase in einer heterologen Wirtszelle exprimiert wird.

2. DNA-Konstrukt nach Anspruch 1, worin die DNA-Sequenz aus dem Chromosom von P. cepacia stammt.

3. DNA-Konstrukt nach Anspruch 2, worin sich die DNA-Sequenz auf dem P. cepacia-Chromosom stromabwärts von einer DNA-Sequenz befindet, die für eine Lipase von ca. 33 kD kodiert.

4. DNA-Konstrukt, das eine erste DNA-Sequenz, die für eine P. cepacia-Lipase oder ein Derivat davon kodiert, und eine zweite DNA-Sequenz, die für einen P. cepacia-Lipase-Modulierungsfaktor, wie er in einem der Ansprüche 1 bis 3 definiert wurde, kodiert, umfaßt.

5. DNA-Konstrukt nach Anspruch 4, worin die erste DNA-Sequenz für eine Lipase von ca. 33 kD kodiert.

6. DNA-Konstrukt nach Anspruch 4, wobei es sich bei der DNA-Sequenz um die in den beigefügten Figg. 2 A-C gezeigte DNA-Sequenz oder eine Modifizierung davon handelt, die für ein Derivat der P. cepacia-Lipase kodiert.

7. DNA-Konstrukt nach Anspruch 4, wobei die für die P. cepacia-Lipase kodierende DNA-Sequenz sich stromaufwärts von der DNA-Sequenz, die für P. cepacia-Lipase-Modulierungsfaktor kodiert, befindet.

8. DNA-Konstrukt nach Anspruch 4, wobei sich die DNA-Sequenz, die für die P. cepacia-Lipase kodiert, stromabwärts von der DNA-Sequenz, die für den P. cepacia-Lipase-Modulierungsfaktor kodiert, befindet.

9. DNA-Konstrukt nach einem der Ansprüche 4 bis 8, wobei die erste und/oder zweite DNA-Sequenz ferner Expressionssignale umfaßt, die gegenüber denen von Pseudomonas heterolog sind.

10. Replizierbarer Expressionsvektor, der eine inserierte DNA-Sequenz trägt, die in den beigefügten Figg. 1 A-C gezeigt ist und für einen Faktor kodiert, der in trans als Modulator der Bildung einer P. cepacia-Lipase wirkt, oder eine Modifizierung dieser DNA-Sequenz, die für ein funktionelles Analogon des P. cepacia-Lipase-Modulierungsfaktors kodiert, wobei der P. cepacia-Lipase-Modulierungsfaktor oder das funktionelle Analogon davon imstande sind, die Lipasebildung zu modulieren, wenn die P. cepacia-Lipase unter Verwendung von Expressionssignalen exprimiert wird, die heterolog gegenüber Pseudomonas sind und/oder wenn die P. cepacia-Lipase in einer heterologen Wirtszelle exprimiert wird.

11. Vektor nach Anspruch 10, wobei die DNA-Sequenz der Definition in Anspruch 2 oder 3 entspricht.

12. Replizierbarer Expressionsvektor, der eine erste inserierte DNA-Sequenz, die für eine P. cepacia-Lipase oder ein Derivat davon kodiert, und eine zweite inserierte DNA-Sequenz, die für einen P. cepacia-Lipase-Modulierungsfaktor, wie er in einem der Ansprüche 1 bis 3 definiert ist, kodiert, trägt.

13. Vektor nach Anspruch 12, wobei die erste DNA-Sequenz für eine Lipase von ca. 33 kD kodiert.

14. Vektor nach Anspruch 13, wobei es sich bei der DNA-Sequenz um die DNA-Sequenz, die in den beigefügten Figg. 2 A-B gezeigt ist, oder eine Modifizierung davon, die für ein Derivat der P. cepacia-Lipase kodiert, handelt.

15. Vektor nach Anspruch 12, worin die erste und die zweite DNA-Sequenz vom gleichen Promotor exprimiert werden.

16. Vektor nach Anspruch 15, wobei sich die erste DNA-Sequenz, die für die P. cepacia-Lipase kodiert, stromaufwärts von der zweiten DNA-Sequenz, die für den P. cepacia-Lipase-Modulierungsfaktor kodiert, befindet.

17. Vektor nach Anspruch 15, wobei sich die erste DNA-Sequenz, die für die P. cepacia-Lipase kodiert, stromabwärts von der zweiten DNA-Sequenz, die für den P. cepacia-Lipase-Modulierungsfaktor kodiert, befindet.

18. Vektor nach Anspruch 12, worin die erste DNA-Sequenz, die für die P. cepacia-Lipase kodiert, und die zweite DNA-Sequenz, die für den P. cepacia-Lipase-Modulierungsfaktor kodiert, jeweils von getrennten Promotoren exprimiert werden.

19. Vektor nach einem der Ansprüche 12 bis 18, worin die erste und/oder die zweite DNA-Sequenz ferner Expressionssignale umfaßt, die gegenüber denjenigen vom Pseudomonas heterolog sind.

20. Wirtszelle, enthaltend ein erstes DNA-Konstrukt, das eine DNA-Sequenz umfaßt, die für eine P. cepacia-Lipase oder ein Derivat davon kodiert, und ein zweites DNA-Konstrukt nach einem der Ansprüche 1 bis 3.

21. Wirtszelle nach Anspruch 20, worin die erste DNA für eine Lipase von ca. 33 kD kodiert.

22. Wirtszelle nach Anspruch 21, wobei es sich bei der DNA-Sequenz des DNA-Konstrukts um die Sequenz, die in den beigefügten Figg. 2 A-B gezeigt ist, oder eine Modifizierung davon, die für ein Derivat der P. cepacia-Lipase kodiert, handelt.

23. Wirtszelle nach einem der Ansprüche 20 bis 22, worin das erste und/oder das zweite DNA-Konstrukt ferner Expressionssignale umfaßt, die gegenüber denjenigen vom Pseudomonas heterolog sind.

24. Wirtszelle, enthaltend ein DNA-Konstrukt nach einem der Ansprüche 4 bis 9.

25. Wirtszelle nach Anspruch 20, die mit einem replizierbaren Expressionsvektor, der das erste DNA-Konstrukt trägt, und mit einem replizierbaren Expressionsvektor nach Anspruch 10 transformiert ist.

26. Wirtszelle nach Anspruch 25, wobei die DNA-Sequenz, die für die P. cepacia-Lipase kodiert, ferner Expressionssignale umfaßt, die gegenüber denjenigen vom Pseudomonas heterolog sind.

27. Wirtszelle nach Anspruch 20, die mit einem replizierbaren Expressionsvektor nach einem der Ansprüche 12 bis 19 transformiert ist.

28. Wirtszelle nach Anspruch 20, die das erste DNA-Konstrukt integriert in ihr Chromosom trägt und die einen replizierbaren Expressionsvektor nach Anspruch 10 enthält.

29. Wirtszelle nach Anspruch 20, die das zweite DNA-Konstrukt integriert in ihr Chromosom trägt und die einen replizierbaren Expressionsvektor enthält, der das erste DNA-Konstrukt enthält.

30. Wirtszelle nach Anspruch 20, die das erste und das zweite DNA-Konstrukt integriert in ihr Chromosom in einer solchen Weise trägt, daß die DNA-Sequenz, die für die P. cepacia-Lipase kodiert, und die DNA-Sequenz, die für den Lipase-Modulierungsfaktor kodiert, jeweils von getrennten Promotoren exprimiert werden.

31. Wirtszelle nach einem der Ansprüche 22 bis 30, wobei es sich um ein Bakterium handelt.

32. Wirtszelle nach Anspruch 31, wobei es sich um ein gram-positives Bakterium handelt.

33. Wirtszelle nach Anspruch 32, wobei das gram-positive Bakterium unter Bacillus subtilis, Bacillus licheniformis und Streptomyces lividans ausgewählt ist.

34. Wirtszelle nach Anspruch 31, wobei es sich um ein gram-negatives Bakterium handelt.

35. Verfahren zur Herstellung einer P. cepacia-Lipase oder eines Derivats davon, wobei das Verfahren das Kultivieren einer Wirtszelle nach einem der Ansprüche 20 bis 34 unter Bedingungen, die für die Bildung der Lipase oder ihres Analogons förderlich sind, und die Gewinnung der Lipase oder ihres Analogons aus dem Kulturmedium umfaßt.

## Revendications

1. Une construction d'ADN, qui comprend une séquence d'ADN telle qu'illustrée sur les figures 1a à 1c annexées, codant pour un facteur qui agit *in trans* comme modulateur de la production d'une lipase de *Pseudomonas cepacia*, ou une modification de cette séquence d'ADN codant pour un analogue fonctionnel du facteur modulateur de la lipase de *P. cepacia*, le facteur modulateur de la lipase de *P. cepacia* ou l'analogue fonctionnel de celui-ci étant capable de moduler la production de lipase lorsque la lipase de *P. cepacia* est exprimée en utilisant des signaux d'expression qui sont hétérologues de *Pseudomonas* et/ou lorsque la lipase de *P. cepacia* est exprimée dans une cellule hôte hétérologue.

2. Une construction d'ADN selon la revendication 1, dans laquelle la séquence d'ADN est dérivée du chromosome de *P. cepacia*.

3. Une construction d'ADN selon la revendication 2, dans laquelle la séquence d'ADN est située sur le chromosome de *P. cepacia* en aval d'une séquence d'ADN codant pour une lipase de ∼33 kD.

4. Une construction d'ADN, qui comprend une première séquence d'ADN codant pour une lipase de *P. cepacia* ou un dérivé de celle-ci, et une seconde séquence d'ADN codant pour un facteur modulateur de la lipase de *P. cepacia* comme défini dans l'une des revendications 1 à 3.

5. Une construction d'ADN selon la revendication 4, dans laquelle la première séquence d'ADN code pour une lipase de ∼33 kD.

6. Une construction d'ADN selon la revendication 4, dans laquelle la séquence d'ADN est celle qui est illustrée sur les figures 2a à 2c annexées ou une modification de celle-ci codant pour un dérivé de la lipase de *P. cepacia*.

7. Une construction d'ADN selon la revendication 4, dans laquelle la séquence d'ADN codant pour la lipase de *P. cepacia* est située en amont de la séquence d'ADN codant pour le facteur modulateur de la lipase de *P. cepacia*.

8. Une construction d'ADN selon la revendication 4, dans laquelle la séquence d'ADN codant pour la lipase de *P. cepacia* est située en aval de la séquence d'ADN codant pour le facteur modulateur de la lipase de *P. cepacia*.

9. Une construction d'ADN selon l'une des revendications 4 à 8, dans laquelle la première et/ou la seconde séquence d'ADN comprennent en outre des signaux d'expression hétérologues de ceux de *Pseudomonas*.

10. Un vecteur d'expression réplicable, qui porte une séquence d'ADN insérée telle qu'illustrée sur les figures 1a à 1c annexées codant pour un facteur qui agit *in trans* comme modulateur de la production d'une lipase de *Pseudomonas cepacia*, ou une modification de cette séquence d'ADN codant pour un analogue fonctionnel du facteur modulateur de la lipase de *P. cepacia*, le facteur modulateur de la lipase de *P. cepacia* ou l'analogue fonctionnel de celui-ci étant capable de moduler la production de lipase lorsque la lipase de *P. cepacia* est exprimée en utilisant des signaux d'expression qui sont hétérologues de *Pseudomonas* et/ou lorsque la lipase de *P. cepacia* est exprimée dans une cellule hôte hétérologue.

11. Un vecteur selon la revendication 10, dans lequel la séquence d'ADN est comme défini dans la revendication 2 ou 3.

12. Un vecteur d'expression réplicable, qui porte une première séquence d'ADN insérée codant pour une lipase de *P. cepacia* ou un dérivé de celle-ci, et une seconde séquence d'ADN insérée codant pour un facteur modulateur de la lipase de *P. cepacia* comme défini dans l'une des revendications 1 à 3.

13. Une construction d'ADN selon la revendication 12, dans laquelle la première séquence d'ADN code pour une lipase de ∼33 kD.

14. Une construction d'ADN selon la revendication 13, dans laquelle la séquence d'ADN est celle qui est illustrée sur les figures 2a à 2b annexées ou une modification de celle-ci codant pour un dérivé de la lipase de *P. cepacia*.

15. Un vecteur selon la revendication 12, dans lequel la première et la seconde séquence d'ADN sont exprimées à partir du même promoteur.

16. Une vecteur selon la revendication 15, dans laquelle la séquence d'ADN codant pour la lipase de *P. cepacia* est située en amont de la séquence d'ADN codant pour le facteur modulateur de la lipase de *P. cepacia*.

17. Une vecteur selon la revendication 15, dans laquelle la séquence d'ADN codant pour la lipase de *P. cepacia* est situéé en aval de la séquence d'ADN codant pour le facteur modulateur de la lipase de *P. cepacia*.

18. Un vecteur selon la revendication 12, dans lequel la première séquence d'ADN codant pour la lipase de *P. cepacia* et la seconde séquence d'ADN codant pour le facteur modulateur de' la lipase de *P. cepacia* sont exprimées chacune à partir d'un promoteur distinct.

19. Un vecteur selon l'une des revendications 12 à 18, dans lequel la première et/ou la seconde séquence d'ADN comportent en outre des signaux d'expression hétérologues de ceux de *Pseudomonas*.

20. Une cellule hôte, contenant une première construction d'ADN comportant une séquence d'ADN codant pour une lipase de *P. cepacia* ou un dérivé de celle-ci, et une seconde construction d'ADN selon l'une des revendications 1 à 3.

21. Une cellule hôte selon la revendication 20, dans laquelle la première construction d'ADN code pour une lipase de ∼33 kD.

22. Une cellule hôte selon la revendication 21, dans laquelle la séquence d'ADN de la construction d'ADN est celle qui est illustrée sur les figures 2a à 2b annexées, ou une modification de celle-ci codant pour un dérivé de la lipase de *P. cepacia*.

23. Une cellule hôte selon l'une des revendications 20 à 22, dans laquelle la première et/ou la seconde constructions d'ADN comprennent en outre des signaux d'expression hétérologues de ceux de *Pseudomonas*.

24. Une cellule hôte, contenant une construction d'ADN selon l'une des revendication 4 à 9.

25. Une cellule hôte selon la revendication 20, qui est transformée avec un vecteur d'expression réplicable portant la première construction d'ADN et avec un vecteur d'expression réplicable selon la revendication 10.

26. Une cellule hôte selon la revendication 25, dans laquelle la séquence d'ADN codant pour la lipase de *P. cepacia* comporte en outre des signaux d'expression hétérologues de ceux de *Pseudomonas*.

27. Une cellule hôte selon la revendication 20, qui est transformée avec un vecteur d'expression réplicable selon l'une des revendication 12 à 19.

28. Une cellule hôte selon la revendication 20, qui porte intégrée dans son chromosome la première construction d'ADN, et qui contient un vecteur d'expression réplicable selon la revendication 10.

29. Une cellule hôte selon la revendication 20, qui porte intégrée dans son chromosome la seconde construction d'ADN et qui contient un vecteur d'expression réplicable contenant la première construction d'ADN.

30. Une cellule hôte selon la revendication 20, qui porte intégrées dans son chromosome la première et la seconde constructions d'ADN, d'une manière telle que la séquence d'ADN codant pour la lipase de *P. cepacia* et la séquence d'ADN codant pour le facteur modulateur de la lipase soient chacune exprimées à partir d'un promoteur distinct.

31. Une cellule hôte selon l'une des revendications 22 à 30, qui est une bactérie.

32. Une cellule hôte selon la revendication 31, qui est une bactérie gram-positive.

33. Une cellule hôte selon la revendication 32, dans laquelle la bactérie gram-positive est choisie dans le groupe formé par *Bacillus subtilis*, *Bacillus licheniformis* et *Streptomyces lividans*.

34. Une cellule hôte selon la revendication 31, qui est une bactérie gram-négative.

35. Un procédé de production d'une lipase de *P. cepacia* ou d'un de ses dérivés, procédé qui comprend la culture d'une cellule hôte selon l'une des revendications 20 à 34 dans des conditions conduisant à la production de la lipase ou d'un analogue, et la récupération de la lipase ou de l'analogue depuis le milieu de culture.
